# EUROPEAN PATENT SPECIFICATION

(11) **EP 4 440 154 B1**
(45) Date of publication and mention of the grant of the patent: **31.12.2025**
(21) Application number: 23165778.4
(22) Date of filing: 31.03.2023
(51) Int. Cl.: H04R 25/00, H04B 5/79, H04L 65/1069, H04W 4/80, A61N 1/36, A61N 1/372, H04L 65/65

(54) **ACQUSITION SEQUENCES OF BINAURAL HEARING SYSTEMS COMPRISING HEARING IMPLANTS**
ERFASSUNGSSEQUENZEN VON BINAURALEN HÖRSYSTEMEN MIT HÖRIMPLANTATEN
SÉQUENCES D'ACQUISITION DE SYSTÈMES AUDITIFS BINAURAUX COMPRENANT DES IMPLANTS AUDITIFS

(43) Date of publication of application: 02.10.2024
(73) Proprietor: GN Hearing A/S, 2750 Ballerup (DK)
(72) Inventor: LØNGAA, Michael, 2750 Ballerup (DK)
(74) Representative: GN Store Nord A/S

(56) References cited:
- EP-A1- 3 657 818
- US-A1- 2012 232 616
- US-A1- 2014 214 123

## Description

The present disclosure relates to acquisition sequences of binaural hearing systems and corresponding binaural hearing systems. The binaural hearing systems may comprise first and second hearing devices, for example head-wearable devices at a user's ears, and first and second hearing implants.

### BACKGROUND OF THE INVENTION

Binaural hearing systems that comprise a pair of cochlea hearing implants may comprise at least four separate devices that are wirelessly connected via at least three bidirectional wireless communication links during normal operation. A first bidirectional wireless communication link must be connected between a first hearing device, typically arranged at, in or on a user's ear, and a first hearing implant such as a cochlea implant. A second bidirectional wireless communication link must be connected between a second hearing device, typically arranged at, in or on the user's opposite ear, and a second hearing implant. Finally, a third bidirectional wireless communication link must be connected between the first and second hearing devices, i.e. a bilateral link, to allow the hearing devices arranged on opposite sides of the user's head to communicate data for example digital audio signals to enable sophisticated binaural processing algorithms and/or synchronize functions of the first and second hearing devices.

The respective acquisitions of the first, second and third bidirectional wireless communication links during an acquisition sequence after power-on of the binaural hearing systems are often time consuming, in particular in an adverse electromagnetic noise environments. This situation will leave the user without audible sound, and thus without auditory awareness, for a prolonged period of time after he/she activates the binaural hearing system until the initial acquisition sequence is completed and normal operation started. Hence, it would be advantageous to rapidly provide the user with audible sound via nerve stimuli by implanted electrode arrays of the first and second cochlea implants quickly after power-on of the binaural hearing system and before the acquisition sequence is completed.

Prior art solutions are known from documents US 2014/214123, US 2012/232616 and EP 3 657 818.

### SUMMARY OF THE INVENTION

A first aspect of the invention relates to a binaural hearing system comprising:
a first hearing device, a second hearing device, a first hearing implant and a second hearing implant; wherein
the first hearing device is connectable to the first hearing implant via a first bidirectional wireless communication link for exchange of first ipsilateral data packets. The second hearing device is connectable to the second hearing implant via a second bidirectional wireless communication link for exchange of second ipsilateral data packets. A bilateral bidirectional wireless communication link is connectable between the first hearing device and second hearing device for exchange of bilateral data packets. The bilateral bidirectional wireless communication link and said first and second bidirectional wireless communication links are configured to operate in accordance with a common communication protocol. The communication protocol may comprise a plurality of consecutive frames each comprising a plurality of time slots such as 4 or more time slots. The common communication protocol comprises an acquisition sequence which comprises:
acquire the first bidirectional wireless communication link using a first processing unit of the first hearing device and acquire the second bidirectional wireless communication link using a second processing unit of the second hearing device. The first and second processing units are configured to acquire the bilateral bidirectional wireless communication link in response to acquisitions of the first and second bidirectional wireless communication links.

After a successful completion of the acquisition sequence, the binaural hearing system preferably enters a normal operation mode where the first hearing device and the first hearing implant are wirelessly connected through the first bidirectional wireless communication link. The second hearing device and the second hearing implant are likewise wirelessly connected through the second bidirectional wireless communication link during normal operation. Finally, the first hearing device and the second hearing device are wirelessly connected through the bilateral bidirectional wireless communication link during normal operation. The common communication protocol may be proprietary and designed for minimal power consumption since each of the first and second hearing devices and each of the first and second hearing implants typically are relatively small battery powered devices or otherwise energized by a power source with limited capacity.

One of the first hearing device and second hearing device is preferably configured as master and the other one as slave for the purpose of acquiring and operating the bilateral bidirectional wireless communication link using their respective first and second processing units as discussed in additional detail below with reference to the appended drawings.

Each of the first and second hearing devices may comprise a head-wearable housing shaped and sized similarly to a traditional hearing aid for example of so-called BTE, ITE, ITC, CIC or RIC types of housings. The housings may be shaped and sized for placement at, or in, a user's left or right ear, for example shaped and sized for placement behind the user's left and right ear pinna. The first and second hearing implants may be configured for placement at the respective sides of the user's skull and configured to supply respective nerve stimulus signals to the user's left and right hearing nerves via implanted electrode arrays.

Each of the first and second bidirectional wireless communication links and the bilateral bidirectional wireless communication link may be based on near-field magnetic coupling, such as NFMI, using respective magnetic coil antennas mounted in the first and second hearing devices. Each of the first and second bidirectional wireless communication links and the bilateral bidirectional wireless communication link may for example use a carrier frequency between 5 and 50 MHz as discussed in additional detail below with reference to the appended drawings.

Each of the first processing unit and second processing unit may comprise a digital signal processor (DSP) and/or a microprocessor such as a software programmable DSP or a software programmable microprocessor. The software programmable DSP or microprocessor may be configured to execute plurality of program instructions configured to implement at least parts of the respective acquisitions of the first and second bidirectional wireless communication links and the bilateral bidirectional wireless communication link in accordance with the common communication protocol. Each of the first processing unit and second processing unit may comprise a dedicated digital state machine configured to handle certain steps of the acquisitions of the first and second bidirectional wireless communication links and the bilateral bidirectional wireless communication link in accordance with the common communication protocol.

According to an embodiment of the common communication protocol and corresponding embodiment of the binaural hearing system, the acquisition sequence comprises:
- maintain at least one of the first bidirectional wireless communication links and second bidirectional wireless communication links during acquisition of the bilateral bidirectional wireless communication link. The maintenance of at least one of the first and second bidirectional wireless communication links during acquisition of the bilateral bidirectional wireless communication link provides the user with sound and general auditory awareness at one side of the user's head until the acquisition sequence is completed and normal operation of the binaural hearing system may be commenced as discussed in further detail below with reference to the appended drawings.

According to an embodiment of the common communication protocol and corresponding embodiment of the binaural hearing system, the acquisition of the first bidirectional wireless communication link comprises:
- transmit a first synchronization marker from the first processing unit to the first hearing implant in a first time slot at the first processing unit,
- monitor the plurality of time slots, at the first hearing implant for the first synchronization marker ,
- sliding the plurality of time slots at the first hearing implant with predetermined time steps between frames of the plurality of consecutive frames using a wrap-around scheme,
- detect the first synchronization marker at the first hearing implant,
- transmit an acknowledge message from the first hearing implant to the first processing unit in second time slot at the first hearing implant,
- synchronize the plurality of time slots at the first hearing implant with the plurality of time slots at the first processing unit based on the first synchronization marker ,
- complete the acquisition of the first bidirectional wireless communication link;
   and
the acquisition of the second bidirectional wireless communication link comprises:
- transmit a second synchronization marker from the second processing unit to the second hearing implant in a first time slot at the second processing unit,
- monitor the plurality of time slots, at the second hearing implant for the second synchronization marker ,
- sliding the plurality of time slots at the second hearing implant with predetermined time steps between frames of the plurality of consecutive frames using a wrap-around scheme,
- detect the second synchronization marker at the second hearing implant,
- transmit an acknowledge message from the second hearing implant to the second processing unit in a second time slot at the second hearing implant,
- synchronize the plurality of time slots at the second hearing implant with the plurality of time slots at the second processing unit based on the second synchronization marker ,
- complete the acquisition of the second bidirectional wireless communication link.

According to an embodiment of the binaural hearing system, the acquisition sequence comprises:
- exchange the first ipsilateral data packets using first and second time slots at the first processing unit of respective frames of the plurality of consecutive frames,
- exchange the second ipsilateral data packets using first and second time slots at the second processing unit of respective frames of the plurality of consecutive frames,
- transmit a third synchronization marker from the first processing unit to the second processing unit in a third time slot at the first processing unit,
- detect the third synchronization marker at the second processing unit,
- synchronize the plurality of time slots at the second processing unit with the plurality of time slots at the first processing unit based on the third synchronization marker ,
- terminate the acquisition sequence,
- enter normal operation of the binaural hearing system.

The common communication protocol may comprise plurality of time slots of each frame such as at least four time slots that enable the first and second hearing devices to exchange the bilateral data packets in the first and second time slots. The plurality of time slots may be non-overlapping time slots. Each of the at least four non-overlapping time slots may have a length between 24 µs and 384 µs. The respective lengths, i.e. duration in time, of the at least four non-overlapping time slots may be identical.

In the first time slot the first processing unit may transmit a bilateral data packet to the second hearing device via the bilateral bidirectional wireless communication link. In a corresponding manner the second hearing device may transmit another bilateral data packet to the first processing unit using the second time slot via the bilateral bidirectional wireless communication link. In the third time slot, each of the first and second processing units may transmit one of the first ipsilateral data packets to their respective hearing implants. In a fourth time slot, each of the first and second hearing implants may transmit one of the second ipsilateral data packets to their respective hearing devices.

In some embodiments of the binaural hearing system at least a subset of the first and second ipsilateral data packets comprises respective digital audio signals or data such as real-time digital audio signals and/or at least a subset of the bilateral data packets comprises respective digital audio data. The respective subsets of the first and second ipsilateral data packets allow the first and second hearing devices to transmit processed sound to the first and second hearing implants. The digital audio signals may, for example, be derived from respective microphone arrangements integrated in housings of the first and second hearing devices.

In one embodiment of the common communication protocol the acquisition of the bilateral bidirectional wireless communication link comprises:
- shift transmission of the second ipsilateral data packets at the second processing unit with one time slot between frames of the plurality of consecutive frames using wrap-around,
- shift transmission of the third synchronization marker with a predetermined time step through the third time slot using wrap-around at the first processing unit.

According to one embodiment of the binaural hearing system the predetermined time step which each of the first, second and third synchronization marker is shifted or slided is less than 5 % of a length of one time slot of the plurality of time slots of the frame.

According to one embodiment of the binaural hearing system the second bidirectional wireless communication link is temporarily broken during the acquisition of the bilateral bidirectional wireless communication link while the first bidirectional wireless communication link is maintained during the acquisition of the bilateral bidirectional wireless communication link. In this embodiment, the acquisition of the bilateral bidirectional wireless communication link may comprise:
- monitor, at the second processing unit, the bidirectional wireless communication link for the second synchronization marker,
- temporarily break the second bidirectional wireless communication link by the second processing unit,
- interrupt the shift of time slots by the second processing unit while maintaining the transmission shift of the synchronization marker by the first processing unit,
- detect by the first processing unit when the second synchronization marker is arranged within the third time slot at the first processing unit,
- detect the second synchronization marker at the second processing unit and transmit an acknowledge message by the second processing unit for example as response,
- re-acquire the second bidirectional wireless communication link before the termination of the acquisition sequence.

In one embodiment of the binaural hearing system the acquisition sequence comprises:
- maintain connection through of the first bidirectional wireless communication link and connection through the second bidirectional wireless communication link during acquisition of the bilateral bidirectional wireless communication link. Since the respective connections of the first and second first bidirectional wireless communication links are established, the exchange of the first ipsilateral data packets and the exchange of the second ipsilateral data packets may provide the user of the binaural hearing system with sound at both ears during the subsequent acquisition of the bilateral bidirectional wireless communication link. That feature is desirable for numerous reasons such as the user's auditory awareness as discussed in further detail below with reference of the appended drawings.

One embodiment of the binaural hearing system, where the connections through both of the first and second bidirectional wireless communication links are maintained during the acquisition of the bilateral bidirectional wireless communication link, comprises:
- shift transmission of the second ipsilateral data packets at the second processing unit by a predetermined time step between frames of the plurality of consecutive frames using wrap-around. The predetermined time step is preferably much shorter than a length of one time slot for example less than 5 % or less than 1 % length of one time slot. The first processing unit monitors the bilateral bidirectional wireless communication link for an acknowledge message transmitted by the second processing unit in response to detection of the third synchronization marker at the second processing unit.

The time step shift of the transmission of the second ipsilateral data packets may comprise:
- advancing the transmission for one or more round trips in a first time direction through the plurality of time slots,
- temporarily interrupt the advancement of the transmission for one or more round trips,
- restart the advancement of transmission in a second time direction through the plurality of time slots for one or more round trips. The first and second time directions are opposite.
- interrupt the advancement of the transmission by the second processing unit in response to transmission of the acknowledge message,
- synchronize the plurality of time slots at the second processing unit with the plurality of time slots at the first processing unit based on the second synchronization marker.

The skilled person will understand that this opposite oriented time sliding or time stepping of the transmissions of the second ipsilateral data packets may serve to compensate for unaligned free-running clock generators and clock signals at the first and second processing units before the bilateral bidirectional wireless communication link is acquired.

According to one embodiment of the binaural hearing system the synchronization marker comprises a unique pair ID for pairing at least one of:
the first hearing device and the first hearing implant,
the first hearing device and the second hearing device; and
the second hearing device and the second hearing implant.

Certain embodiments of the binaural hearing system utilize three different unique pair IDs in the synchronization marker to avoid erroneous pairing of devices of the binaural hearing system as discussed in further detail below with reference of the appended drawings.

A second aspect relates to a binaural hearing system comprising
a first hearing device connected to a first hearing implant via a first bidirectional wireless communication link for exchange of first ipsilateral data,
a second hearing device connected to a second hearing implant via a second bidirectional wireless communication link for exchange of second ipsilateral data,
the first hearing device connected to the second hearing device via a bilateral bidirectional wireless communication link connecting for exchange of bilateral data; wherein
said bilateral bidirectional wireless communication link and said first and second bidirectional wireless communication links are configured to operate in accordance with a common communication protocol which comprises a plurality of consecutive frames each comprising a plurality of time slots;
said common communication protocol comprising an acquisition sequence which comprises:
   acquire a first connection through the first bidirectional wireless communication link using a first processing unit of the first hearing device,
   acquire a second connection through the second bidirectional wireless communication link using a second processing unit of the second hearing device, wherein the first processing unit is configured to acquire a third connection through the bilateral bidirectional wireless communication link in response to the acquisition by the first processing unit of the first hearing device of the first connection through the first bidirectional wireless communication link and in response to the acquisition by the second processing unit of the second hearing device of the second connection through the second bidirectional wireless communication links.

The content of each of the first, second and third synchronization markers may be defined by the common communication protocol and identical except for unique pair IDs as discussed in further detail below with reference of the appended drawings.

A third aspect of the invention relates to an acquisition sequence, e.g. an computer-implemented acquisition method, for acquiring respective wireless connections between a first hearing device and a first hearing implant, between a second hearing device and a second hearing implant and between the first hearing device and the second hearing device.

The acquisition sequence comprising:
- acquire a first bidirectional wireless communication link between the first hearing device and the first hearing implant,
- exchange first ipsilateral data packets through the first bidirectional wireless communication link,
- acquire a second bidirectional wireless communication link between the second hearing device and the second hearing implant,
- exchange second ipsilateral data packets through the second bidirectional wireless communication link; and in response to acquisitions of the first and second bidirectional wireless communication links:
   - acquire, in response to acquisitions of the first and second bidirectional wireless communication links, a bilateral bidirectional wireless communication link between the first and second hearing devices,
   - exchange bilateral data packets between the first hearing device and the second hearing device.

One embodiment of the acquisition sequence comprises:
- exchange the first ipsilateral data packets using first and second time slots at the first processing unit of respective frames of the plurality of consecutive frames,
- exchange the second ipsilateral data packets using first and second time slots at the second processing unit of respective frames of the plurality of consecutive frames,
- transmit a third synchronization marker from the first processing unit to the second processing unit in a third time slot at the first processing unit,
- detect the synchronization marker at the second processing unit,
- synchronize the plurality of time slots at the first processing unit with the plurality of time slots at the second processing unit based on the third synchronization marker.

### BRIEF DESCRIPTION OF THE DRAWINGS

In the following exemplary embodiments of the invention are described in more detail with reference to the appended drawings, wherein:

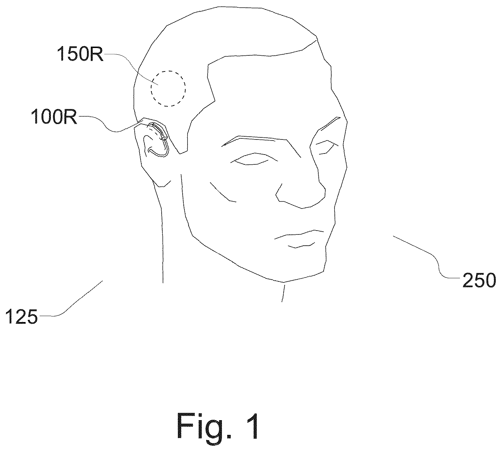

FIG. 1 schematically illustrates an exemplary binaural hearing system in accordance with the invention mounted on user's head,

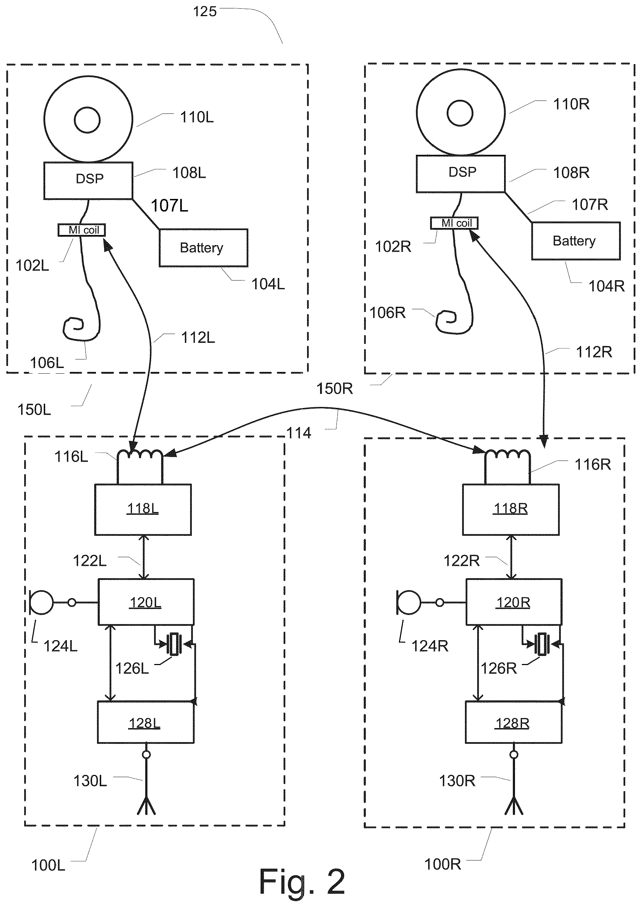

FIG. 2 shows respective schematic block diagrams of first and second hearing devices and associated first and second hearing implants of the exemplary binaural hearing system,

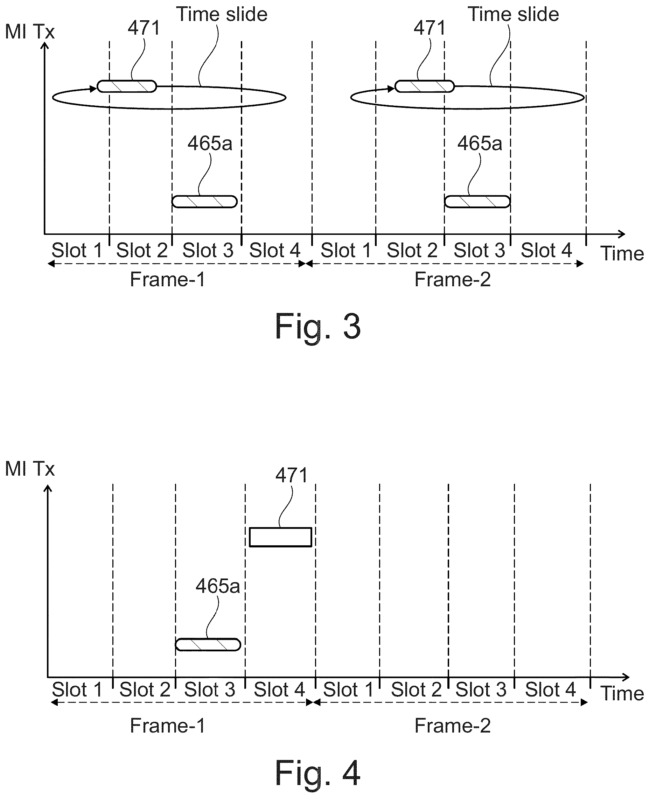

FIG. 3 schematically illustrates transmission of a first synchronization marker from the first hearing device to the first hearing implant during acquisition of the first bidirectional wireless communication link according to the embodiments of the exemplary binaural hearing system,

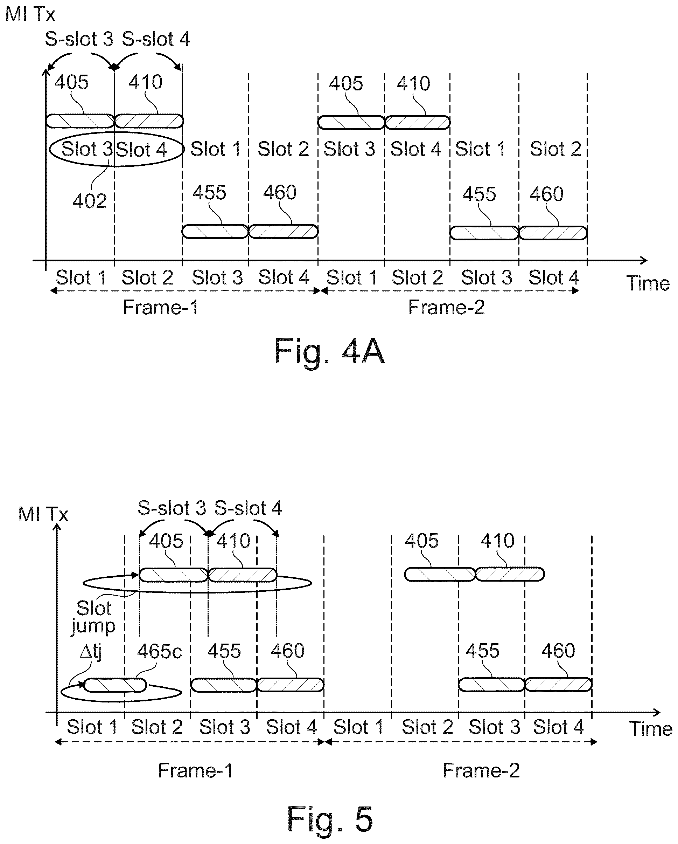

FIG. 4 schematically illustrates detection of the first synchronization marker by the first hearing implant and its response by transmission of an acknowledge message during acquisition of the first bidirectional wireless communication link according to embodiments of the exemplary binaural hearing system,
FIG. 4A schematically illustrates exchange of respective ipsilateral data packets between the first hearing device and the first hearing implant and between the second hearing device and the second hearing implant following the acquisition of the first and second bidirectional wireless communication links in accordance with embodiments of the exemplary binaural hearing system,
FIG. 5 schematically illustrates exchange of respective ipsilateral data packets between the first hearing device and the first hearing implant and between the second hearing device and the second hearing implant during acquisition of a bilateral bidirectional wireless communication link in accordance with a first embodiment,

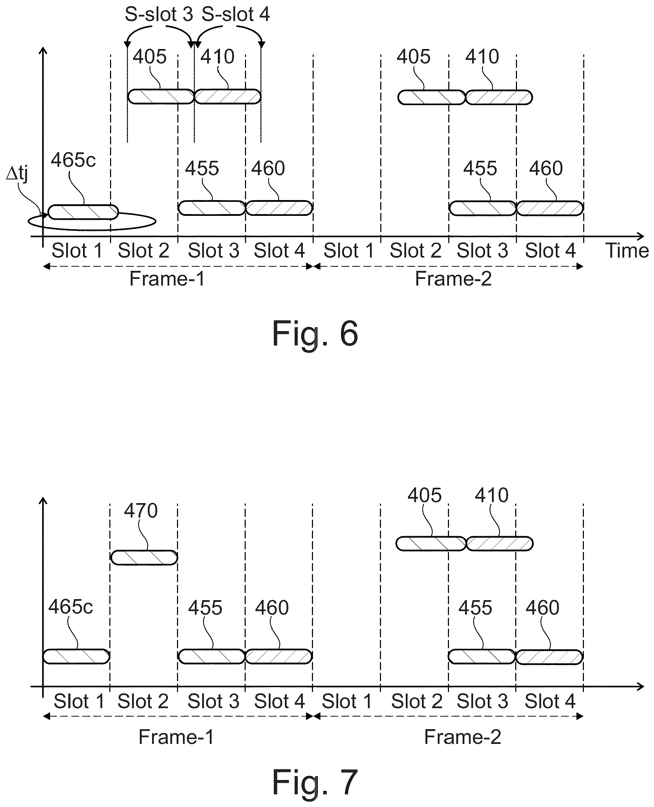

FIG. 6 schematically illustrates the use of a sliding synchronization marker during the acquisition of the bilateral bidirectional wireless communication link in accordance with the first embodiment of the exemplary binaural hearing system,
FIG. 7 schematically illustrates the use of the sliding synchronization marker during the acquisition of the bilateral bidirectional wireless communication link in accordance with the first embodiment of the exemplary binaural hearing system,

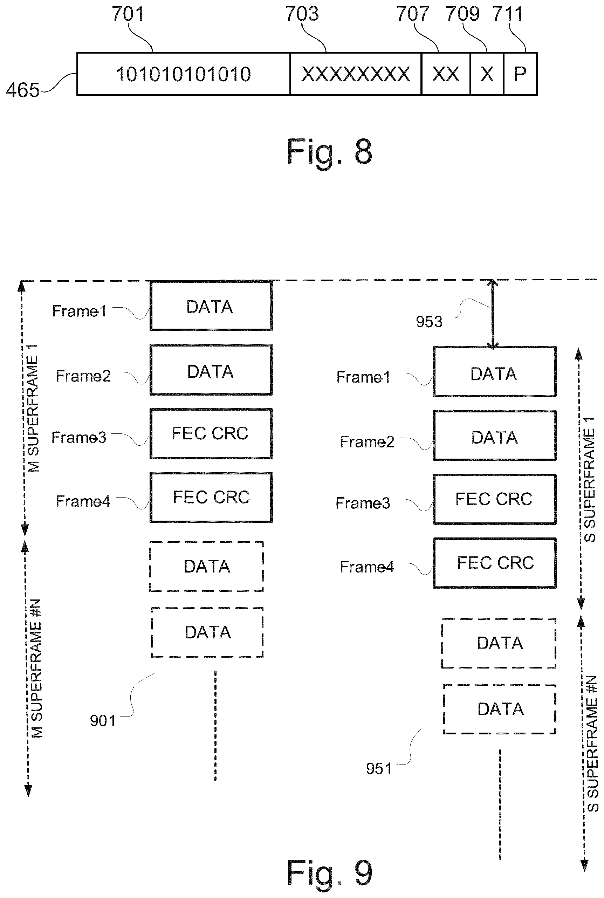

FIG. 8 illustrates features of an exemplary synchronization marker utilized during the acquisition of the bilateral bidirectional wireless communication link in accordance with the first and second embodiments of the exemplary binaural hearing system,
FIG. 9 illustrates exemplary super-frame structures each comprising a plurality of frames of the ipsilateral data packets and bilateral data packets in accordance with the first and second embodiments of the exemplary binaural hearing system,

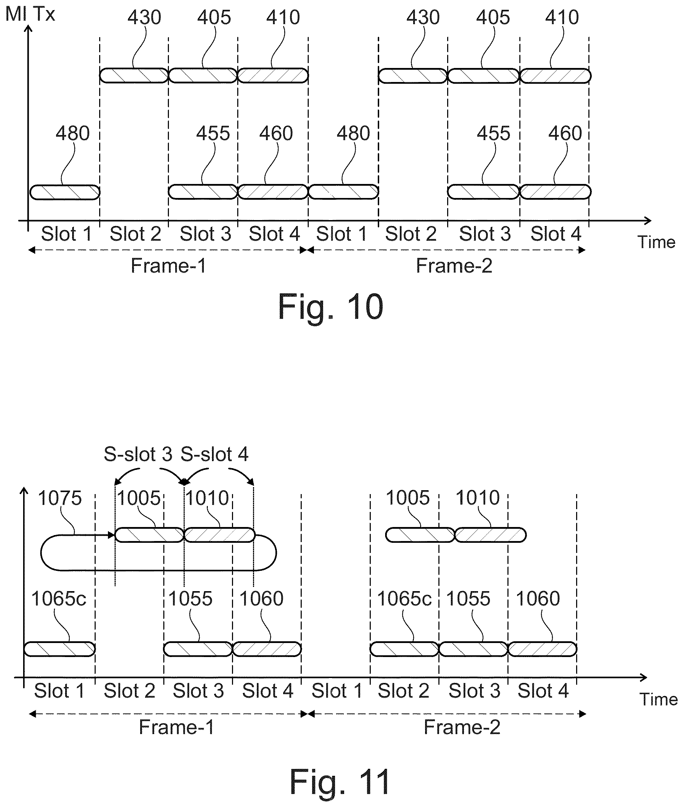

FIG. 10 schematically illustrates exchange of ipsilateral data packets and bilateral data packets during normal operation, i.e. payload mode, of the bilateral hearing system after completion of an acquisition sequence in accordance with the first and second embodiments of the exemplary binaural hearing system,
FIG. 11 schematically illustrates the exchange of respective ipsilateral data packets between the first hearing device and the first hearing implant and between the second hearing device and the second hearing implant during acquisition of a bilateral bidirectional wireless communication link in accordance with a second embodiment of the exemplary binaural hearing system,

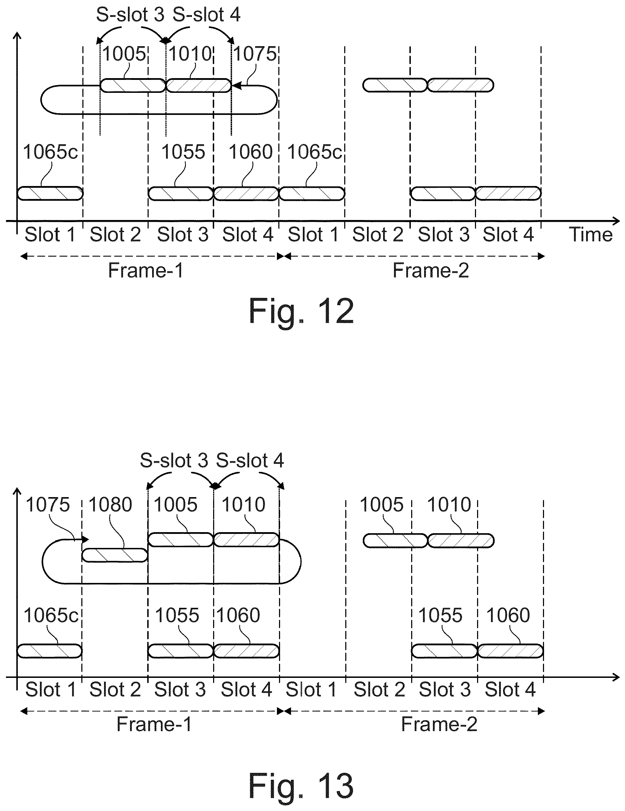

FIG. 12 schematically illustrates the exchange of respective ipsilateral data packets between the first hearing device and the first hearing implant and between the second hearing device and the second hearing implant during the acquisition of the bilateral bidirectional wireless communication link in accordance with the second embodiment of the exemplary binaural hearing system,
FIG. 13 schematically illustrates the exchange of respective ipsilateral data packets between the first hearing device and the first hearing implant and between the second hearing device and the second hearing implant during the acquisition of the bilateral bidirectional wireless communication link in accordance with the second embodiment of the exemplary binaural hearing system; and

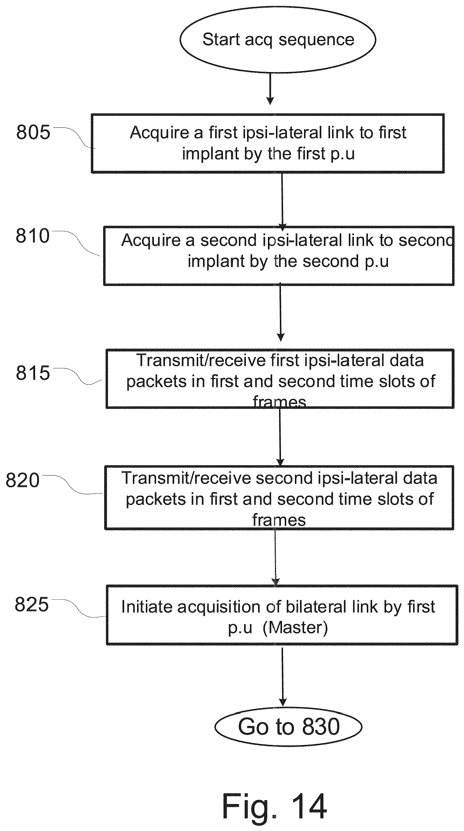

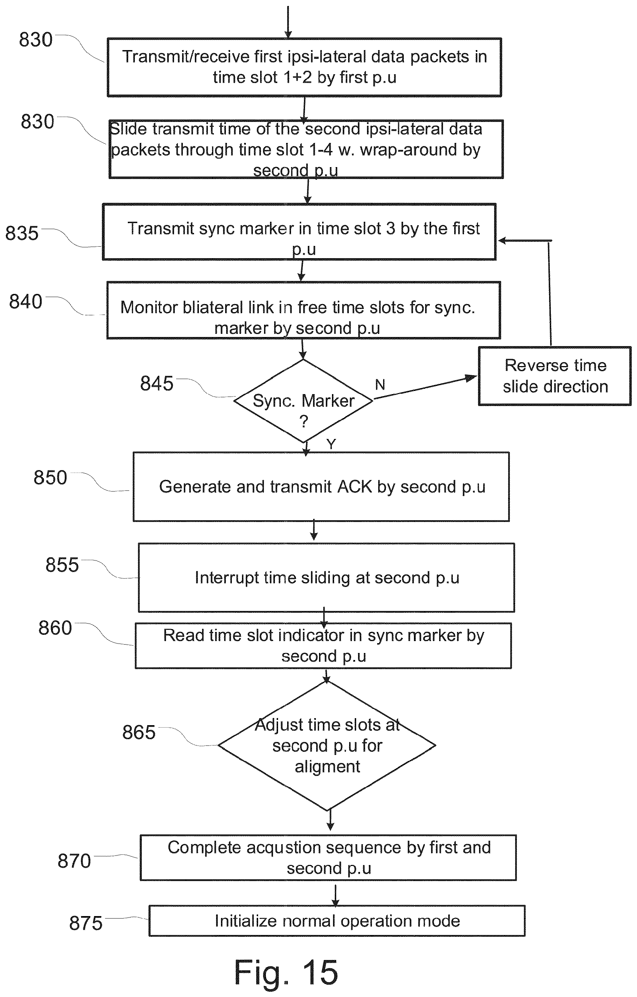

FIG. 14 and FIG. 15 are flow charts illustrating respective processing steps carried out by the respective processing units of the first and second hearing devices during acquisition of the bilateral bidirectional wireless communication link according to the second embodiment of the binaural hearing system.

### DETAILED DESCRIPTION OF EMBODIMENTS

In the following various exemplary embodiments of the present hearing system are described with reference to the appended drawings. The skilled person will understand that the accompanying drawings are schematic and therefore merely show details which are essential to understanding of the invention. Other details may have been left out or simplified for brevity and clarity. Like reference numerals refer to like elements throughout the drawings. Like elements will, thus, not necessarily be described in detail with respect to each drawing.

FIG. 1 schematically illustrates a first hearing device 100L and a first hearing implant 150L of an exemplary binaural hearing system 125. The binaural hearing system 125 further comprises a second hearing device 100L and a second hearing implant 150L both arranged at the opposite side of the user's head 250 and therefore not visible. The first hearing implant 150L is adapted for surgical implantation in the user's skull and therefore invisible from the outside when implanted. The second hearing implant 150R is arranged in a corresponding manner at the opposite side of the user's head 250.

FIG. 2 is a block diagram of the binaural hearing system 125. The first hearing device 100L is connectable to the first hearing implant 150L via a first bidirectional wireless communication link 112L ("first link") for exchange of first ipsilateral data packets. The second hearing device 100L is likewise connectable to the second hearing implant 150L via a second bidirectional wireless communication link 112L ("second link") for exchange of second ipsilateral data packets.

The binaural hearing system 125 further comprises a bilateral bidirectional wireless communication link 114 ("bilateral link", or "third link") that is connectable between the first hearing device 100L and second hearing device 100R for exchange of bilateral data packets. The first hearing device 100L and second hearing device 100R are preferably configured to use the respective magnetic coil antennas 116L, 116R for wireless communication with the first hearing implant 150L and second hearing implant 150R, respectively.

The bilateral link 114, first link 112L and second link 112R are preferably configured to utilize, i.e. operate in accordance with, a common data communication protocol. The common data communication protocol comprises a plurality of consecutive time frames that each comprises a plurality of time slots holding the first and second ipsilateral data packets and the bilateral data packets. The plurality of first and second ipsilateral data packets and the bilateral data packets are structured, transmitted and received in accordance with the common data communication protocol as discussed in further detail below.

The skilled person will understand that certain types of data packets such as synchronization markers and acknowledge messages may be wirelessly transmitted between the first hearing device 100L and the second hearing device 100R, between the first hearing device 100L and the first hearing implant 150L and between the second hearing device 100R and the second hearing implant 150R before the respective completions of the acquisitions of the bilateral link 114, first link 112L and second link 112R.

The bilateral data packets exchanged by the bilateral link 114 may comprise digital audio samples and control information transmitted by respective ones of the first and external hearing devices 100L, 100R. Each of the first and second hearing devices 100L, 100R may transmit digital audio samples and control data to the first and second hearing implant 150L, 150R, respectively. Each of the first and second hearing implants 150L, 150R may additionally be configured to transmit respective control data to the first and second hearing devices 100L, 100R via the first link 112L and second link 112R. In the latter embodiment both the first link 112L and the second link 112R are adapted for bidirectional data transfer.

Each of the first link 112L, the second link 112R and the bilateral link 114 may in certain embodiments of the invention be based on, or comprise, near-field magnetic coupling, such as NFMI links. The first link 112L may accordingly comprise a magnetic coil antenna 116R of the first hearing device 100L and a magnetic coil antenna 102R of the first hearing implant 150L. The second link 112R may likewise comprise a magnetic coil antenna 116L of the second hearing device 100L and a magnetic coil antenna 102L of the second hearing implant 100L. Each of first link 112L, the second link 112R and the bilateral link 114 may for example utilize a carrier frequency between 5 MHz and 50 MHz, such as between 9 MHz and 27 MHz for transmissions of the first and second ipsilateral data packets and the bilateral data packets.

The first hearing implant 150L comprises a first control unit 108L that is connected to the magnetic coil antenna 102L for receipt and processing of the first ipsilateral data packets transmitted by the first hearing device 100L. The first control unit 108L is further configured to transmit the first ipsilateral data packets to the first hearing device 100L via the first link 112L. In some embodiments, a first processing unit 108L of the first hearing implant 150L may comprise a digital signal processor (DSP) and/or a microprocessor. Certain embodiments of the first hearing implant 150L may comprise a rechargeable battery assembly 104L. The rechargeable battery assembly 104R is configured for receiving electrical power from a receiver (Rx) charging coil 110L during dedicated recharging operations or sessions. The receiver (Rx) charging coil 110L may be energized by an appropriately positioned external, i.e. outside the user's skull, transmitter (Tx) charging coil (not shown) of a charging device (not shown) during the dedicated recharging sessions. This allows wireless transfer of power to the rechargeable battery assembly 104L. The rechargeable battery assembly 104L is preferably coupled to a power supply input of an implant processor 108L to energize the latter as schematically indicated by a power line or wire 107R. The implant processor 108L may comprise a microprocessor for example software programmable microprocessor.

The first hearing implant 150L further comprises an electrode array 106L for insertion into a cochlea of the user during implantation. The electrode array 106L may be electrically coupled to the first processing unit 108L that supplies suitable electrode stimuli signals to the electrode array 106L to stimulate the user's cochlea nerve. The skilled person will understand that these electrode stimuli signals may be generated by the first processing unit 108L and derived by the latter based on the first ipsilateral data packets, in particular digital audio signals or data embedded or held in the first ipsilateral data packets. The skilled person will appreciate that corresponding functions, structures and features of the second hearing implant 150R may be largely identical to those of the first hearing implant 150L.

The block diagram on FIG. 2 further illustrates an exemplary embodiment of the first hearing device 100L. The first hearing device 100L comprises the magnetic coil antenna 116L which is electrically connected to a transceiver 118L. The transceiver 118R is configured to repeatedly switch between Tx and Rx modes to modulate and demodulate incoming and outgoing data of the first ipsilateral data packets and bilateral data packets. The transceiver 118L may be configured to convert the first ipsilateral data packets and bilateral data packets to a format understood by a first processing unit 120L of the first hearing device 100L. In some embodiments, the first processing unit 120L may comprise a digital signal processor (DSP) and/or a microprocessor such as a software programmable DSP or a microprocessor.

The first hearing device 100L further comprises one or more microphones 124L that are coupled to an appropriate audio interface of the first processing unit 120L. The first processing unit 120L may be configured to execute a suitable operating system. The operating system may be configured to manage various hardware and software resources of the first hearing device 100L such as handling of the common communication protocol, computation of monaurally or bilaterally beamformed microphone signals, hearing loss compensation processing of the microphone signal(s), the first wireless data communication interface 118L, certain memory resources etc. The operating system may schedule tasks for efficient use of hearing device resources and may further include accounting software for cost allocation, including power consumption, processor time, memory locations, wireless transmissions, and other resources. The operating system may be stored in and retrieved from a non-volatile memory (not shown), e.g. flash memory or EEPROM, of the processing unit 120L.

Each of the first and second hearing devices 100L,100R may comprise a housing of a type that is well-known from the hearing aid industry like so-called BTE, ITE, ITC, CIC or RIC housing types. These housing types are shaped and sized for placement at, or in, the user's ear. The first hearing device 100L further comprises a system clock generator 126L that is configured to supply clock signals to various digital logic circuits and components of the device 100L including the first processing unit 120R as schematically illustrated. A nominal value of a clock frequency of the system clock generator 126R may lie between 2 MHz and 64 MHz such as between 10 MHz and 50 MHz. The first processing unit 120R may be configured to derive respective lengths of certain time slots and time frames of the common communication protocol of the first link 112L and the bilateral link 114 as discussed in additional detail below.

The first hearing device 100L may additionally comprise a second, optional, wireless communication interface 128L and RF antennal 130L configured to jointly communicate through a second wireless communication link (not shown). The second wireless communication link and interface may be configured to operate in the 2.4 GHz industrial scientific medical (ISM) band and may be compliant with a Bluetooth LE standard. This second wireless communication link may provide convenient data connectivity to various types of portable communication devices like smartphones, mobile phones, tablets and personal computers etc. due to the industry standard compatible nature of the second wireless communication link. Various types of control data and audio data may be transmitted from the portable communication device to the first hearing device 100L and vice versa. The second hearing device 100L may for the same purpose comprise a similar optional second wireless communication interface 128L and RF antenna 130L as illustrated.

In the following disclosure the first hearing device 100L of the binaural hearing system 125 is assigned as master during execution of the common communication protocol while the second hearing device 100R is configured as a slave. The skilled person will understand that a system clock signal of the master may control timing of transmissions of respective data packets through the first link 112L, the second link 112R and the bilateral link 114 at least after the acquisitions of the first link 112L, second link 112R and bilateral link 114.

FIG. 3 schematically illustrates transmission of a first synchronization marker 465a from the master processing unit 120L of the first or master hearing device 100L to the first hearing implant 150L during acquisition of the first link 112L according to embodiments of the common communication protocol of the exemplary binaural hearing system 125.

A "time slot" is the shortest time division of the bilateral bidirectional wireless communication link and first and second bidirectional wireless communication links as defined by the common communication protocol. One data packet may for example be transmitted in one time slot.

A "frame" is a unit of data that comprises a predefined number of time slots as defined by a communication protocol.

"Connectable" shall mean that the wireless communication link in question is configured to establish a wireless connection between specified devices after acquisition of the wireless communication link in question.

A "bidirectional wireless communication link" is a wireless communication link that supports transmission of data packets or data messages from a first device to a second device and transmission of data packets or data messages from the second device to the first device.

"Ipsilateral data packets" are data packets exchanged between a hearing device and a hearing implant arranged at the same side of a user's head.

A "hearing implant" shall mean that portion of a cochlear implant that is located inside the user's skull.

During the acquisition of the first link 112L the master processing unit 120L operates as a master device towards the first hearing implant 150L. The master processing unit 120L may start to repeatedly transmit the first synchronization marker 465a to the first hearing implant 150L using one of a plurality of time slots, e.g. time slots 1-4, of each frame, Frame-1, Frame-2 etc., of a plurality of consecutive frames. The indicated time slots 1-4 are referenced to the master processing unit 120L. Time slot 3, at the master processing unit 120L, is selected for transmission of the first synchronization marker 465a in the present example as illustrated and made in accordance with the common communication protocol. The first hearing implant 150L resides concurrently in a receipt mode such that the first hearing implant 150L monitors the first link 112L to detect arrival of the first synchronization marker 465a. However, the respective time slots 1-4 at the master processing unit 120L and the first hearing implant 150L are most likely more or less unaligned or non-synchronous at the current acquisition step of the acquisition sequence caused by free-running clock generators and clock signals at the master processing unit 120L and first hearing implant 150L. As illustrated by FIG. 3 where the first hearing implant 150L searches for a first synchronization marker 465a, preferably have a first unique pair ID as discussed further below, in a time slot 471 at the first hearing implant 150L. The exemplary time slot 471 is shifted or misaligned with about one-fourth of the length of one time slot relative to the time slots at the master processing unit 120L. This misalignment of time slots prevents the first hearing implant 150L from detecting the first synchronization marker 465a detecting the first synchronization marker 465c because the latter extends across two adjacent time slots at the second hearing device 100R.

The first hearing implant 150L therefore advances or slides its time slots 1-4, such as the exemplary time slot 471, with predetermined time steps between frames of the plurality of consecutive frames using a wrap-around scheme as schematically indicated by "Time slide" arrow. The predetermined time step is preferably much shorter than a length of one time slot such as less 5 % or less than 1 % of the length of one time slot. The first hearing implant 150L continues to slide its time slots 1-4 until the latter time slots are sufficiently aligned with the time slots 1-4 at the master processing unit 120L to detect receipt of the first synchronization marker 465a at the second implant 150L. The second control unit 108R of the second implant 150L may check the unique pair ID of the received synchronization marker to ensure it is transmitted from a paired device, i.e. the master hearing device 100L in the present situation. The second control unit 108R may reject the received synchronization marker if it is not transmitted by the paired device. In the latter situation, the second control unit 108R may thereafter continue the search for the first synchronization marker 465a. When the first synchronization marker 465a is detected, the first hearing implant 150L responds by reading a slot indicator bit or field of the first synchronization marker 465a as discussed in further detail below. The first hearing implant 150L may proceed, based on that time slot identification, to align time slots at the first hearing implant 150L, i.e. at the first implant processor 108L, with the corresponding time slots at the master processing unit 120L of the first hearing device 100L. Hence, synchronize data packet exchange or communication between the first hearing implant 150L and the master processing unit 120L through the first link 112L. The first implant processor 108L may proceed by shifting to a transmission mode and transmit an acknowledge message 471 (ACK) to the master processing unit 120L in time slot 4 at the master processing unit 120L as schematically illustrated by FIG. 4... Thereafter, the master processing unit 120L may respond by terminating the acquisition process of the first link 112L because the latter is now appropriately connected between the master processing unit 120 and the first hearing implant 150L in accordance with the first and second embodiments of the common communication protocol of the binaural hearing system 125. The master processing unit 120L and the first hearing implant 150L are accordingly ready to exchange the first ipsilateral data packets 455, 460 using time slots 3 and 4 as outlined in further detail below.

The skilled person will appreciate that the acquisition of the second link 112R of the exemplary binaural hearing system 125 may follow a corresponding scheme to create an appropriate connection between the slave processing unit 120R and the second hearing implant 150R. Preferably, the slave processing unit 120R operates as a master device towards the second hearing implant 150L. However, a second synchronization marker, that has different unique pair ID for that of the first synchronization marker 465a, is preferably utilized by the slave processing unit 120R and the second hearing implant 150L for the acquisition of the second link 112R. The difference between the unique pair IDs (ID 703 on FIG. 8) of the first and second synchronization markers ensures that only the appropriate pair of master and slave devices 120R, 150R or 120L, 150L are connected to each other. Otherwise, communication crosstalk between the master side and slave side, for example between the first hearing device 100L and the second hearing implant 150R, could lead to erroneous pairing. The first and second synchronization markers may be distinguished by the unique pair IDas discussed in further detail below.

FIG. 4A illustrates in schematic form exchange of first and second ipsilateral data packets 455, 460, 405, 410, respectively between the master and slave hearing devices 100L, 100R and associated hearing implants 150L, 150R in accordance with the first embodiment of the common communication protocol of the binaural hearing system 125. The illustrated exchange of the first and second ipsilateral data packets 455, 460, 405, 410, respectively, is carried out after the master processing unit 120L has acquired the first bidirectional wireless communication link to make the wireless connection, e.g. first connection, between the master hearing device 100L and the first hearing implant 150L as outlined in detail above. The slave processing unit 120R has independently, and for example substantially simultaneously, acquired the second bidirectional wireless communication link 112R as outlined in detail above to make the wireless connection, e.g. a second connection, between the slave hearing device 100R and the second hearing implant 150R. During the acquisition of the first link 112L the master processing unit 120L is preferably configured to operate as a master device towards the first hearing implant 150L. The respective acquisitions of the first link 112L and second link 112R that establish respective connections through the first and second links 112L, 112R may be viewed as an intermediate step of the acquisition sequence in accordance with the first and second embodiments of the common communication protocol. The subsequent acquisition of the bilateral link 114 that establishes wireless connection between the master processing unit 120L and the slave processing unit 120R can be viewed as a final step of the acquisition sequence.

In connection with the intermediate step of the acquisition sequence, FIG. 4A illustrates the exchange of the first ipsilateral data packets 455, 460 between the master processing unit 120L and the first hearing implant 150L. In a similar manner the second ipsilateral data packets 405, 410 are exchanged between the slave processing unit 120R and the second hearing implant 150R. In a frame, like frame-1, the master processing unit 120L transmits the first ipsilateral data packets 455, 460 to the first hearing implant 150L using respective predetermined time slots, such as slots 3 and 4, or any other pair, of the illustrated time slots 1-4, at the master side. The master processing unit 120L transmits the first ipsilateral data packets 455, 460 in respective ones of the plurality of consecutive frames such as frame-1, frame-2 etc. The master side of the binaural hearing system 125 may be seen as a combination of the master hearing device 100L and the first hearing implant 150L. The slave side of the binaural hearing system 125 may be seen as a combination of the slave hearing device 100R and first hearing implant 150R.

The slave processing unit 120R exchanges the second ipsilateral data packets 405, 410 with the second hearing implant 150R using respective predetermined time slots, such as time slots 3 and 4, at the slave side of the binaural hearing system 125 following a similar transmission scheme and data packet structure at the master side.

The skilled person will understand that other embodiments of the common communication protocol may specify more than four time slots per frame such as between 5 and 16 time slots. Irrespective of the actual number of time slots, such as at least four time slots, these are preferably non-overlapping time slots. Each time slot may have a length between 24 µs and 384 µs for example depending a number of bits of at least some of the exchanged data packets. The length of each of the time slots may be identical. Each of the first ipsilateral data packets 455, 460 and the second ipsilateral data packets 405, 410 may comprise identical number of bits for example between 4 bits and 96 bits such as between 6 bits and 24 bits. The number of bits in a particular packet may depend on its type of content, i.e. digital audio signals, control information or a combination of both.

At least some of the first ipsilateral data packets 455, 460 and at least some of the second ipsilateral data packets 405, 410 comprise digital audio signals and/or control information. The control information may be held in a packet header and the digital audio signals held in a packet payload. The digital audio signals may be perceptually encoded to reduce the amount of audio data in the messages for transmission. The respective digital audio signals of the first ipsilateral data packets 455, 460 and second ipsilateral data packets 405, 410 may be generated by, or derived, from respective microphone arrangements of the master and slave external hearing devices 100L,100R for example integrated with respective housings of the master and slave external hearing devices 100L,100R. The respective digital audio signals of the first ipsilateral data packets 455, 460 and second ipsilateral data packets 405, 410 may alternatively be derived from remote microphone arrangements wirelessly coupled to the master and slave external hearing devices 100L, 100R. The sound may comprise speech, noise or any mixture thereof, for example sound present in a user's external environment or sound streamed sound from an audio enabled portable device.

In some embodiments of the binaural hearing system 125, those first and second ipsilateral data packets 460, 410 that are exchanged in respective time slots 4 are transmitted from the first and second hearing implants 150L, 150R to their respective master and slave processing units 120L, 120R in accordance with the common communication protocol. In that embodiment, the master processing unit 120L operates in receipt mode in time slot 4 and monitors the first link 112L to detect the first ipsilateral data packet 455. The slave processing unit 120R operates in a corresponding manner for receipt of the data packets 410 transmitted by the second hearing implant 150R in time slot 4 at the slave side. The respective data packets 460, 410 transmitted by the first and second hearing implants 150L 150R in respective time slots 4 may exclusively comprise control information, i.e. no digital audio signals. The control information may include certain types of data protocol parameters such as pair ID information, error detecting codes etc. associated with the first hearing implant 150L and/or the second hearing implant 150R.

In some embodiments of the binaural hearing system 125 both of the first ipsilateral data packets 455, 460 in time slots 3 and 4, respectively, are transmitted from the master processing unit 120L to the first hearing implant 150L in accordance with the common communication protocol. In that embodiment, the master processing unit 120L operates in transmission mode (Tx) in both time slots 3 and 4 while the first hearing implant 150L operates in receipt mode (Rx) and monitors the first link 112L to detect each of the first ipsilateral data packet 455, 460. The slave processing unit 120R and second hearing implant 150R operate in a corresponding manner for transmission and receipt of the second ipsilateral data packets 405, 410 at the slave side. Further, in some embodiments of the common communication protocol, the type of content of the data packets 455, 460, 405, 410 differ from frame to frame of the consecutive frames. The consecutive frames may optionally be organised in so-called super-frames which each comprises a plurality of individual frames, such as between 4 and 32 individual frames, to provide different types of content of the data packets 455, 460, 405, 410 as discussed in additional detail below.

The skilled person will appreciate that the illustrated exemplary timing on FIG. 4A of the respective transmissions of the first and second ipsilateral data packets 455, 460, 405, 410 through the first and second links 112L, 112R are worst-case unaligned or non-synchronous where the time slots 3 and 4 at the slave side are aligned with time slots 1 and 2 at the master side, as indicated by reference symbol 402 and S-slot 3 and S-slot-4, after acquisition of the first and second links 112L, 112R. In this worst-case situation there are not any unoccupied time slots in a frame for data transmission during the acquisition of the bilateral link 114 or for exchange of the bilateral data packets.

The skilled person will appreciate that after the acquisitions of the first and second links 112L, 112R the alignment between the plurality of time slots, e.g. slots 1- 4, at the master side and the corresponding time slots at the slave side will be random. Hence, plurality of time slots may be misaligned with any amount for example by 0.5 slot, 1 time slot, 1.5 time slot and even the illustrated worst case situation with a misalignment of exactly two time slots out of the four time slots 1-4. This random time slot alignment is caused by the free-running clock generators and clock signals at the master and slave processing units 120L, 120R. In practice the clock signals of the master and slave processing units 120L, 120R will differ slightly in frequency and phase even when the clock generators are nominally identical. Hence, the time slots at the master side and slave side are most likely more or less unaligned or non-synchronous after the intermediate step of the acquisition sequence of the hearing system.

FIG. 5 illustrates in schematic form the first embodiment of the acquisition of the bilateral link 114 by the master processing unit 120L and slave processing unit 120R. The acquisition of the bilateral link 114 is carried out in response to, and hence after, the above-outlined acquisition of the first link 112L and the acquisition of the second link 112R and further in accordance with the acquisition sequence of the common communication protocol. The slave processing unit 120R may enter into a listening mode or state in the unoccupied time slots 1 and 2 at the slave processing unit 120R after the acquisition of the second link 112R. The master processing unit 120L may enter into a transmit mode or state in the unoccupied time slots 1 and 2 at the master processing unit 120L after the acquisition of the first link 112R. The master processing unit 120L may thereafter start to transmit a third synchronization marker 465c in the unoccupied time slots via the bilateral link 114 for detection by the slave processing unit 120R as described in additional detail below.

As schematically illustrated, the data packets 405, 410 in time slots 3 and 4, respectively, at the master side and the time slots 3 and 4 at the slave side are shifted or misaligned with about two-thirds of the length of one time slot. This situation is illustrated by indications of time slots 3 and 4, s-slot 3, s-slot 4, at the slave processing unit 120R taking as reference the time slots 1-4 at the master side depicted on the time-axis. Since the first and second links 112L, 112R are established, the exchange of the first ipsilateral data packets 455, 460 at the master side and the exchange of the second ipsilateral data packets 405, 410 at the slave side, through the connections made by the first and second links 112L, 112R, provide the user of the hearing system with sound in at least one, and possibly, both ears during the subsequent acquisition of the bilateral link 114. The latter property is desirable because the transmission of the sound in at least one of the user's ears provides the user with basic auditory awareness to hear speech and other sound signals in the surrounding environment. The subsequent acquisition of the bilateral link 114, and subsequent exchange of the bilateral data packets during connection, may further enhance the user's listening comfort and improve speech intelligibility by applying sophisticated binaural processing algorithms like beamforming, noise reduction etc. by the master and slave processing units 120L, 120R.

For the purpose of acquiring the bilateral link 114, the slave processing unit 120R is configured to shift, e.g. advance, transmission of the second ipsilateral data packets 405, 410 at the slave processing unit 120R with one time slot at every predetermined number of frames of the plurality of consecutive frames for example every 8^{th} 16^{th}, 32nd or 64^{th} frame. The time slot shift of transmission of the second ipsilateral data packets 405, 410 uses a wrap-around scheme as illustrated by the depicted arrow "slot jump". Hence, the shift of time slots at the second processing unit 120L frees different time slots over time at the second processing unit 120 for possible transmission or receipt of the bilateral data packets. As illustrated on FIG. 5, time slot 1 of frame-1 and frame-2 etc. is unoccupied or vacant at the current moment in time.

The master processing unit 120L may be configured to concurrently generate and transmit the third synchronization marker 465c to the slave processing unit 120R through the bilateral link 114 using unoccupied time slots 1 and 2 at the master processing unit 120L. The master processing unit 120L is additionally configured to shift, e.g. slide, transmission of the third synchronization marker 465c with a predetermined time step through time slots 1 and 2 from frame to frame using a wrap-around scheme as schematically illustrated by arrow Δtj. The wrap-around scheme means that that the sliding of the third synchronization marker 465c jumps back to the start of time slot 1 when the end of time slot 4 is reached by the sliding of the third synchronization marker 465c.

The predetermined time step is preferably much smaller than the slot length and hence also the frame length. The predetermined time step Δtj may be 250 ns for a frame length of 192 µs. The same ratio between the predetermined time step and the frame length may be used for alternative frame lengths for example frame lengths between 100 and 400 µs. The corresponding length of a time slot may be about 48 µs for a frame length of 192 µs where the common communication protocol allocates four time slots of the same length per frame as the present embodiment.

FIG. 6 illustrates in schematic form additional steps of the acquisition of the bilateral link 114 by the master processing unit 120L and slave processing unit 120R according to the previously disclosed first embodiment of the binaural hearing system 125. The slave processing unit 120R is configured to monitor the bidirectional wireless communication link 114 for the third synchronization marker 465c transmitted by master processing unit 120L as outlined above. In response to detection of the third synchronization marker 465c the slave processing unit 120R interrupts the time slot hopping or shifting discussed above in connection with FIG. 5. The slave processing unit 120R further responds by temporarily disconnecting or breaking the second link 112R. Accordingly, the second ipsilateral data packets 405, 410 are temporarily no longer transmitted at the slave side of the hearing system 125 which leaves all four time slots unoccupied by the second ipsilateral data packets 405, 410 at the slave side as schematically depicted on FIG. 7. However, the master processing unit 120L continues to exchange the second ipsilateral data packets 455, 460 in time slots 3 and 4 whereby sound may be transmitted to the user's master side hearing implant 150L in an uninterrupted manner also during the temporary breakage of the second link 112R during the present embodiment of the acquisition sequence and hearing system 125.

The master processing unit 120L preferably continues to transmit the third synchronization marker 465c while sliding the third synchronization marker 465c through time slots 1 and 2 using the wrap-around scheme until such time that the third synchronization marker 465c is arranged within time slot 1 and transmitted therein. The slave processing unit 120R monitor for and detects the third synchronization marker 465c which may follow the above-outlined two-step scheme where the slave processing unit 120R initially recognizes the predetermined search sequence 701 and thereafter performs the "long packet search" to detect the entire content of the third synchronization marker 465c. The slave processing unit 120R thereafter reads the slot indicator 709 of the third synchronization marker 465c and aligns the time slots 1-4 at the slave processing unit 120R with the corresponding time slots at the master processing unit 120L to synchronize the time slots, and hence the frames as well, at the master side and slave side of the binaural hearing system 125. The slave processing unit 120R proceeds by transmitting an acknowledge message (ACK) 470 back to the master processing unit 120L via the bilateral link 114 as schematically depicted on FIG. 8. The master processing unit 120L responds by completing the acquisition of the bilateral link 114 and the slave processing unit 120R proceeds by reestablishing the connection through the second link 112R which has been temporarily interrupted. Thereafter, the master processing unit 120L responds by terminating the acquisition sequence because each of the first link 112L, the second link 112R and the bilateral link 114 is now appropriately connected and all links are time aligned or synchronized in accordance with the first embodiment of the common communication protocol of the binaural hearing system 125,

In response to completion of the acquisition sequence, the master processing unit 120L, the slave processing unit 120R, the first hearing implant 150L and the second hearing implant 120R enter normal operation, i.e. a "payload mode", where the first and second ipsilateral data packets 455, 460, 405, 410, respectively, are exchanged in time slots 3 and 4 and the bilateral data packets 480, 430 are exchanged in time slots 1 and 2, respectively, of the consecutive frames as schematically illustrated on FIG. 10.

Some embodiments of the common communication protocol comprise that the master processing unit 120L is configured to respond to receipt of the acknowledge message 470 by starting a countdown sequence before the payload mode is started. The countdown sequence may comprise an exchange of several rounds, e.g. 2, 3 or 4 rounds, of the third synchronization marker 465c and accompanying acknowledge message 470 (ACK) between the master processing unit 120L and the slave processing unit 120R before the termination of the acquisition sequence and entry into payload mode. This countdown sequence may further verify the reliability of the bilateral link 114.

After the acquisition of the bilateral link 114, the slave processing unit 120R reconnects the temporarily disconnected second link 112R so that the exchange of the second ipsilateral data packets 405, 410 between the slave hearing device 100L and first hearing implant 150L is restarted.

The skilled person will appreciate that the uninterrupted operation of at least one of the first and second links 112L, 112R during the entire acquisition sequence is a favourable property. This uninterrupted operation is carried out by the on-going exchange of the first and second ipsilateral data packets 455, 460, 405, 410, respectively, so that sound pick-up at first and second hearing devices 100L, 100R is processed and conveyed to the user of the binaural hearing system 125.

FIG. 8 illustrates an exemplary synchronization marker 465 that may be utilized for the respective acquisitions of the bilateral bidirectional wireless communication link 114, the first link 112L and the second link 112R. The exemplary synchronization marker 465 preferably comprises unique pair ID 703. The unique pair ID 703 may be a coder or number and comprise between 8 bits and 32 bits. A first unique pair ID may be utilized to pair the master hearing device 100L and the first hearing implant 150L to each other. The first unique pair ID may be stored in a memory of the master processing unit 120L and further stored in a memory of the first control unit 108L of the first hearing implant 150L. The first unique pair ID may for example be stored during manufacture of the master hearing device 100L or later in connection with a fitting of the binaural hearing system 125 to the user. The first unique pair ID may be stored in a similar manner in the memory of the first control unit 108L. A second unique pair ID may be utilized to pair the slave hearing device 100L and the second hearing implant 150L to each other. The second unique pair ID may be stored in the memory of the master processing unit 120L and further stored in a memory of the second control unit 108R of the second hearing implant 150R. The second unique pair ID may for example be stored in the master processing unit 120L and the second control unit 108R in a similar manner to the first unique pair ID 703 as outlined above.

A third unique pair ID may be utilized to pair the slave hearing device 100L and the master hearing device 100L to each other. The third unique pair ID may be stored in the memory of the master processing unit 120L and further stored in the memory of the slave processing unit 120R. The third unique pair ID may for example be stored in the slave processing unit 120R and the master processing unit 120L in a similar manner to the first unique pair ID as outlined above.

The different unique pair IDs 703 ensures that only the appropriate pair of devices are connected to each other during the respective acquisitions of the first link 112L, second link 112R and bilateral link 114. Otherwise, unintended crosstalk between the devices, for example between the first and second second hearing implants 150L, 150R of the binaural hearing system 125 could lead to erroneous pairing of devices. The unique pair IDs in a similar manner prevent unintended crosstalk and erroneous pairing of devices of two different but nearby located bilateral hearing systems. The synchronization marker 465 can be viewed as a special type of data packet and may have the same length and number of bits as at least some of the first and second ipsilateral data packets. The synchronization marker 465 may further comprise a predetermined search sequence 701, for example a predetermined binary pattern like 10101010 or equivalently 01010101. The latter is known to both the master processing unit 120L and the slave processing unit 120R a priori in a similar manner to the unique pair IDs. The predetermined binary pattern may be received and recognized by the slave processing unit 120R to detect the synchronization marker 465 in a currently unoccupied time slot at the slave processing unit 120R.

The synchronization marker 465 may comprise an optional wrap-around countdown number. The latter may be utilized by the master processing unit 120L and slave processing unit 120R for providing an additional layer of safety once the slave processing unit 120R and master processing unit 120L have acknowledged each other through the bilateral link as described in additional detail below. A slot indicator 709 tells the slave processing unit 120R in which time slot at the master side the synchronization marker 465 is arranged to allow the slave processing unit 120R in aligning its time slots with the corresponding time slots at the master processing unit 120L. The slave processing unit 120R may for example determine a time offset between the already known time slots at the slave processing unit 120R and the corresponding time slots at the master processing unit 120R for alignment. Since, the length of a time slot, e.g. 48 µs, is known to the slave processing unit 120R the time offset can indicate slot alignment.

A "long packet search" may be started by the slave processing unit 120R in the time slot where the search sequence 701 was last observed. The long packet search is utilized by the slave processing unit 120R to read and evaluate the entire data content of the third synchronization marker 465c once the predetermined binary pattern is recognized or detected by the slave processing unit 120R. The third synchronization marker 465c may comprise a parity bit 711 or any other type of suitable error detection and/or correction code. The slave processing unit 120R may utilize the error detection and/or correction code to evaluate data integrity of the synchronization markers in well-known manners.

FIG. 9 illustrates an exemplary super-frame structure comprising where each super-frame comprises a plurality of individual frames, such as between 4 and 32 individual frames, of the plurality of consecutive frames in accordance with one embodiment of the common communication protocol. The frames at the master side of the binaural hearing system are structured in consecutive super-frames 901 and the super-frames at the slave side of the binaural hearing system are structured in consecutive super-frames 951. Each frame such as frame-1 may carry the same type of data, for example digital audio signals, which means that all data packets of the frame in question hold the same type of data e.g. digital audio signals, control information or error-correction codes like CRC and/or forward error-correction codes (FEC) etc. The super-frames at the master and slave sides may be misaligned in time, i.e. a time off-set, as schematically illustrated by an off-set arrow 953. The slave processing unit 120R may be configured to detect this time off-set and transmit it to the master processing unit 120L which proceeds to align the super-frames 901, 951 between the master side and slave side.

FIGS. 11, 12 and 13 illustrate various steps of an acquisition of a bilateral bidirectional wireless communication link between the master and slave external hearing devices 100L, 100R and associated hearing implants 150L, 150R of the exemplary binaural hearing system 125 in accordance with a second embodiment of the common communication protocol.

The illustrated exchange on FIG. 11 of the first and second ipsilateral data packets 1055, 1060, 1005, 1010, respectively, is carried out after the master processing unit 120L has acquired a connection, e.g. a first connection, to the first hearing implant 150L through the first link 112L in accordance with the second embodiment of the binaural hearing system 125 operating in accordance with a corresponding embodiment of the common communication protocol. The operation of the second embodiment of the binaural hearing system 125 follows the steps of the above-described acquisition of the first link 112L in accordance with the first embodiment of the binaural hearing system 125 and common communication protocol. The slave processing unit 120R has independently, and for example substantially simultaneously, acquired the connection, e.g. a second connection, to the second hearing implant 150R through the second link 112R which acquisition may follow the steps of the above-described acquisition of the first link 112L in accordance with the first embodiment. After acquisitions of the first link 112L and second link 112R, the subsequent exchange of respective data packets between the master processing unit 120L and the first hearing implant 150L and between the slave processing unit 120R and the second hearing implant 150L may be similar to the one described above in the first embodiment of the common communication protocol.

However, as illustrated by FIG. 11, the subsequent acquisition of the bilateral link 114 carried out by the master processing unit 120L and the slave processing unit 120R in accordance with the second embodiment comprises *inter alia* a transmission of a first synchronization marker 1065c in a fixed predetermined time slot at the master processing unit 120L, e.g. slot 1, other than time slots already occupied by the first ipsilateral data packets 1055, 1060 as illustrated. The first synchronization marker 1065cc may be transmitted in each frame, or a subset of frames e.g. every 4^{th} or 8^{th} frame etc., of a plurality of consecutive frames, i.e. frame-1, frame-2 etc.

Concurrently, the slave processing unit 120R advances or slides transmission of the second ipsilateral data packets 1005, 1010 with predetermined time steps between frames of the plurality of consecutive frames using a wrap-around scheme. The predetermined time step is preferably shorter than a length of one time slot such as less 5 % or less than 1 % of the length of one time slot. The slave processing unit 120R may advance or slide the transmission of the second ipsilateral data packets 1005, 1010 for two or more round trips through time slots 1-4 in a first time direction such as from slots 2 and 3 towards slots 3 and 4. This advancement of the transmission of the second ipsilateral data packets 1005, 1010 is schematically depicted on FIG. 11 by wrap-around arrow 1075. The wrap-around scheme means that that the sliding of the time slots at slave processing unit 120R, such as S-slot 3 and S-slot-4, jumps back to time slot 1 when the end of time slot 4 is reached by the sliding of the second ipsilateral data packets 1005, 1010.

The slave processing unit 120R may thereafter temporarily interrupt advancing the transmission of the second ipsilateral data packets 1005, 1010 for a predetermined time period but continue to transmission of the second ipsilateral data packets 1005, 1010. Subsequently, the slave processing unit 120R may advance the transmission of the second ipsilateral data packets 1005, 1010 for two or more round trips through time slots 1-4 in a second time direction, opposite to the first time direction, such as from slots 2 and 3 towards slots 1 and 2. This opposite advancement or sliding of the transmission of the second ipsilateral data packets 1005, 1010 is schematically depicted on FIG. 12 by oppositely oriented arrow 1075. The slave processing unit 120R further monitors the bilateral link 114 during the bi-directional time wise advancement of the second ipsilateral data packets 1005, 1010 to detect receipt of the first synchronization marker 1065c. The skilled person will understand that this opposite sliding of the transmission of the second ipsilateral data packets 1005, 1010 is advantageous to compensate for the previously discussed free-running clock generators and clock signals at the master and slave processing units 120L, 120R and guarantee alignment when the clock signals at the master and slave processing units 120L, 120R differ in frequency. As discussed above, the time slots at the master side and slave side are most likely more or less unaligned or non-synchronous after the intermediate step of the acquisition sequence of the binaural hearing system 125 as illustrated by FIG. 12 where the time slots 3 and 4 at the master side and time slots 3 and 4 at the slave side are shifted or misaligned with about two-thirds of the length of one time slot.

The master processing unit 120L monitors the bilateral link 114 for an acknowledge message (ACK) 1080 transmitted by the slave processing unit 120R in time slot 2 in response to detection of the third synchronization marker 1065c transmitted by the master processing unit 120L in time slot 1 as schematically depicted on FIG. 13. The slave processing unit 120R may check the unique pair ID of a received synchronization marker to ensure it is transmitted from a paired device, i.e. the master hearing device 100L in the present situation. The slave processing unit 120R may reject the synchronization marker if it is not transmitted by the paired device. If the slave processing unit 120R fails to detect the proper unique pair ID of the received synchronization marker 1065c during the bi-directional time slide process of the second ipsilateral data packets 1005, 1010, the slave processing unit 120R may be configured to re-start and repeat the time slide process until the third synchronization marker 1065c is detected. The slave processing unit 120R finally interrupts the time slide transmission of process in response to its transmission of the acknowledge message (ACK) 1080 that confirms to the master processing unit 120L the slave processing unit's 120R detection of the synchronization marker 1065c. The synchronization marker 1065c preferably comprises the previously discussed slot indicator 709 where the master processing unit 120L indicates to the slave processing unit 120R which time slot at the master side that holds the third synchronization marker 465c so that the slave processing unit 120R may align its time slots with the corresponding time slots at the master processing unit 120L.

The master processing unit 120L responds to receipt of the acknowledge message (ACK) 1080 by completing the acquisition of the bilateral link 114 and the slave processing unit 120R proceeds in a similar manner. Thereafter, the master processing unit 120L proceeds by terminating the acquisition sequence because each of the first link 112L, the second link 112R and the bilateral link 114 are now appropriately connected and corresponding time slots of all links are time aligned or synchronized in the binaural hearing system 125 in accordance with the common communication protocol. In response to completion of the acquisition sequence, the master processing unit 120L, the slave processing unit 120R, the first hearing implant 150L and the second hearing implant 120R each commences normal operation, i.e. a "payload mode", where the first and second ipsilateral data packets 1055, 1060, 1005, 1010, respectively, are exchanged in time slots 3 and 4 and the bilateral data packets 480, 430 are exchanged in time slots 1 and 2, respectively, of the consecutive frames as schematically illustrated on FIG. 10.

FIG. 14 and FIG. 15 are flow charts illustrating processing steps 805 - 875 carried out by the first and second processing units (first p.u and second p.u) of the first and second hearing devices in connection with the above-outlined acquisition of the bilateral bidirectional wireless communication link 114 according to the second embodiment of the binaural hearing system 125.

## Claims

1. A binaural hearing system comprising:
a first hearing device, a second hearing device, a first hearing implant and a second hearing implant; wherein
the first hearing device is connectable to the first hearing implant via a first bidirectional wireless communication link for exchange of first ipsilateral data packets,
the second hearing device is connectable to the second hearing implant via a second bidirectional wireless communication link for exchange of second ipsilateral data packets,
a bilateral bidirectional wireless communication link is connectable between the first hearing device and second hearing device for exchange of bilateral data packets; wherein
said bilateral bidirectional wireless communication link and said first and second bidirectional wireless communication links are configured to operate in accordance with a common communication protocol which comprises a plurality of consecutive frames each comprising a plurality of time slots;
said common communication protocol comprising an acquisition sequence which comprises:
acquire the first bidirectional wireless communication link using at least a first processing unit of the first hearing device,
acquire the second bidirectional wireless communication link using at least a second processing unit of the second hearing device, wherein the first and second processing units are configured to acquire the bilateral bidirectional wireless communication link in response to acquisitions of the first and second bidirectional wireless communication links.

2. A binaural hearing system according to claim 1, wherein the acquisition sequence comprises:
- maintain at least one of the first bidirectional wireless communication link and second bidirectional wireless communication link during acquisition of the bilateral bidirectional wireless communication link.

3. A binaural hearing system according to claim 1 or 2, wherein:
the acquisition of the first bidirectional wireless communication link comprises:
- transmit a first synchronization marker from the first processing unit to the first hearing implant in a first time slot at the first processing unit,
- monitor the plurality of time slots, at the first hearing implant for the synchronization marker,
- slidie the plurality of time slots at the first hearing implant with predetermined time steps between frames of the plurality of consecutive frames using a wrap-around scheme,
- detect the first synchronization marker at the first hearing implant,
- synchronize the plurality of time slots at the first hearing implant with the plurality of time slots at the first processing unit based on the first synchronization marker,
- complete the acquisition of the first bidirectional wireless communication link,
- transmit an acknowledge message from the first hearing implant to the first processing unit in second time slot at the first processing unit;
and
the acquisition of the second bidirectional wireless communication link comprises:
- transmit a second synchronization marker from the second processing unit to the second hearing implant in a first time slot at the second processing unit,
- monitor the plurality of time slots, at the second hearing implant for the second synchronization marker,
- sliding the plurality of time slots at the second hearing implant with predetermined time steps between frames of the plurality of consecutive frames using a wrap-around scheme,
- detect the second synchronization marker at the second hearing implant,
- synchronize the plurality of time slots at the second hearing implant with the plurality of time slots at the second processing unit based on the synchronization marker,
- transmit an acknowledge message from the second hearing implant to the second processing unit in a second time slot at the second processing unit,
- complete the acquisition of the second bidirectional wireless communication link.

4. A binaural hearing system according to any of claims 1-3,
wherein the acquisition sequence comprises:
- exchange the first ipsilateral data packets through the first bidirectional wireless communication link using the first and second time slots at the first processing unit of respective frames of the plurality of consecutive frames,
- exchange the second ipsilateral data packets through the second bidirectional wireless communication link using first and second time slots at the second processing unit of respective frames of the plurality of consecutive frames,
- transmit a third synchronization marker from the first processing unit to the second processing unit in a third time slot at the first processing unit,
- detect the third synchronization marker at the second processing unit,
- synchronize the plurality of time slots at the second processing unit with the plurality of time slots at the first processing unit based on the third synchronization marker,
- transmit an acknowledge message from the second processing unit to the first processing unit in a second time slot at the first processing unit,
- complete the acquisition sequence,
- enter normal operation of the binaural hearing system.

5. A binaural hearing system according to claim 3 or 4, wherein the acquisition of the bilateral bidirectional wireless communication link comprises:
- shift transmission of the second ipsilateral data packets at the second processing unit with one time slot between frames of the plurality of consecutive frames using wrap-around,
- shift transmission of the synchronization marker with a predetermined time step through the third time slot using wrap-around at the first processing unit.

6. A binaural hearing system according to claim 5, wherein the predetermined time step is less than 5 % of a length of one time slot of the plurality of time slots of the frame.

7. A binaural hearing system according to any of claims 2-6, wherein the acquisition of the bilateral bidirectional wireless communication link comprises:
- monitor, at the second processing unit, for the third synchronization marker,
- temporarily break the second bidirectional wireless communication link by the second processing unit,
- interrupt the shift of time slots by the second processing unit while maintaining the transmission shift of the third synchronization marker by the first processing unit,
- detect by the first processing unit when the third synchronization marker is arranged within the third time slot at the first processing unit,
- detect the third synchronization marker at the second processing unit and transmit an acknowledge message by the second processing unit,
- re-acquire the second bidirectional wireless communication link before the termination of the acquisition sequence.

8. A binaural hearing system according to any of claims 1-4, wherein the acquisition sequence comprises:
- maintain connection through the first bidirectional wireless communication link and maintain connection through the second bidirectional wireless communication link during acquisition of the bilateral bidirectional wireless communication link.

9. A binaural hearing system according to claim 8, wherein the acquisition of the bilateral bidirectional wireless communication link comprises:
- shift transmission of the second ipsilateral data packets at the second processing unit by a predetermined time step between frames of the plurality of consecutive frames using a wrap-around scheme, wherein said predetermined time step is shorter than a length of one time slot,
- monitor, by the first processing unit, the bilateral bidirectional wireless communication link for an acknowledge message transmitted by the second processing unit in response to detection of the third synchronization marker at the second processing unit.

10. A binaural hearing system according to claim 9, wherein the time step shift of the transmission of the second ipsilateral data packets comprises:
- advancing the transmission for one or more round trips in a first time direction through the plurality of time slots,
- temporarily interrupt the advancement of the transmission for one or more round trips,
- restart the advancement of transmission in a second time direction through the plurality of time slots for one or more round trips;
wherein the first and second time directions are opposite;
- interrupt the advancement of the transmission by the second processing unit in response to transmission of the acknowledge message,
- synchronize the plurality of time slots at the second processing unit with the plurality of time slots at the first processing unit based on the synchronization marker.

11. A binaural hearing system according to any of claims 2-10, wherein each of the first synchronization marker, second synchronization marker and third synchronization marker at least comprises:
- a predetermined binary sequence,
- a time slot indicator for indicating which time slot of the plurality of time slots at the first processing unit that holds the synchronization marker.

12. A binaural hearing system according to any of claims 2-11, wherein each frame of the plurality of consecutive time frames comprises at least four non-overlapping time slots.

13. A binaural hearing system according to claim 11 or 12, wherein a length of each of the at least four non-overlapping time slots is identical.

14. A binaural hearing system according to any of the preceding claims, wherein at least a subset of the first and second ipsilateral data packets comprises respective digital audio data such as real-time digital audio signals; and/or at least a subset of the bilateral data packets comprises respective digital audio data such as real-time digital audio signals.

15. A binaural hearing system according to any of claims 3-14, wherein each of the first synchronization marker, second synchronization marker and third synchronization marker comprises a unique pair ID such as a unique code or number, for pairing at least one of:
the first hearing device and the first hearing implant,
the first hearing device and the second hearing device; and
the second hearing device and the second hearing implant.

16. An acquisition sequence in accordance with a common communication protocol for acquiring respective connections between a first hearing device and a first hearing implant, between a second hearing device and a second hearing implant and between the first hearing device and the second hearing device; said acquisition sequence comprising:
- acquire a first bidirectional wireless communication link between the first hearing device and first hearing implant,
- exchange first ipsilateral data packets through the first bidirectional wireless communication link,
- acquire a second bidirectional wireless communication link between the second hearing device and second hearing implant,
- exchange second ipsilateral data packets through the second bidirectional wireless communication link; and in response to acquisitions of the first and second bidirectional wireless communication links:
- acquire a bilateral bidirectional wireless communication link between the first and second hearing devices, in response to acquisitions of the first and second bidirectional wireless communication links,
- exchange bilateral data packets between the first hearing device and the second hearing device.

## Patentansprüche

1. Ein binaurales Hörsystem, umfassend:
ein erstes Hörgerät, ein zweites Hörgerät, ein erstes Hörimplantat und ein zweites Hörimplantat; wobei
das erste Hörgerät über eine erste bidirektionale drahtlose Kommunikationsverbindung zum Austausch erster ipsilateraler Datenpakete mit dem ersten Hörimplantat verbunden werden kann,
das zweite Hörgerät über eine zweite bidirektionale drahtlose Kommunikationsverbindung zum Austausch zweiter ipsilateraler Datenpakete mit dem zweiten Hörimplantat verbunden werden kann,
eine bilaterale bidirektionale drahtlose Kommunikationsverbindung zwischen dem ersten und dem zweiten Hörgerät zum Austausch bilateraler Datenpakete besteht;
wobei
die bilaterale bidirektionale drahtlose Kommunikationsverbindung sowie die erste und zweite bidirektionale drahtlose Kommunikationsverbindung so konfiguriert sind, dass sie gemäß einem gemeinsamen Kommunikationsprotokoll arbeiten, das eine Vielzahl aufeinanderfolgender Frames umfasst, die jeweils eine Vielzahl von Zeitschlitzen enthalten;
Das genannte gemeinsame Kommunikationsprotokoll umfasst eine Erfassungssequenz, die Folgendes beinhaltet: Erfassung der ersten bidirektionalen drahtlosen Kommunikationsverbindung mit mindestens einer ersten Verarbeitungseinheit des ersten Hörgeräts,
Erfassung der zweiten bidirektionalen drahtlosen Kommunikationsverbindung mit mindestens einer zweiten Verarbeitungseinheit des zweiten Hörgeräts, wobei die erste und die zweite Verarbeitungseinheit so konfiguriert sind, dass sie die bilaterale bidirektionale drahtlose Kommunikationsverbindung als Reaktion auf die Erfassung der ersten und zweiten bidirektionalen drahtlosen Kommunikationsverbindung erfassen.

2. Ein binaurales Hörsystem nach Anspruch 1, wobei die Erfassungssequenz Folgendes umfasst:
- Aufrechterhaltung mindestens einer der beiden bidirektionalen drahtlosen Kommunikationsverbindungen während der Erfassung der bilateralen bidirektionalen drahtlosen Kommunikationsverbindung.

3. Ein binaurales Hörsystem nach Anspruch 1 oder 2, wobei der Aufbau der ersten bidirektionalen drahtlosen Kommunikationsverbindung Folgendes umfasst:
- Senden eines ersten Synchronisationsmarkers von der ersten Verarbeitungseinheit an das erste Hörimplantat in einem ersten Zeitschlitz der ersten Verarbeitungseinheit,
- Überwachen der Zeitschlitze am ersten Hörimplantat auf den Synchronisationsmarker,
- Verschieben der Zeitschlitze am ersten Hörimplantat mit vorbestimmten Zeitschrittweiten zwischen den Frames der mehreren aufeinanderfolgenden Frames mittels eines Wrap-Around-Schemas,
- Erkennen des ersten Synchronisationsmarkers am ersten Hörimplantat,
- Synchronisieren der Zeitschlitze am ersten Hörimplantat mit den Zeitschlitzen an der ersten Verarbeitungseinheit basierend auf dem ersten Synchronisationsmarker,
- Abschließen des Aufbaus der ersten bidirektionalen drahtlosen Kommunikationsverbindung,
- Senden einer Bestätigungsnachricht vom ersten Hörimplantat an die erste Verarbeitungseinheit im zweiten Zeitschlitz der ersten Verarbeitungseinheit ; Die Herstellung der zweiten bidirektionalen drahtlosen Kommunikationsverbindung umfasst folgende Schritte: - Senden eines zweiten Synchronisationsmarkers von der zweiten Verarbeitungseinheit an das zweite Hörimplantat in einem ersten Zeitschlitz der zweiten Verarbeitungseinheit,
- Überwachen der Zeitschlitze des zweiten Hörimplantats auf den zweiten Synchronisationsmarker,
- Verschieben der Zeitschlitze des zweiten Hörimplantats mit vorbestimmten Zeitschrittweiten zwischen den Frames der mehreren aufeinanderfolgenden Frames mittels eines Wrap-Around-Verfahrens,
- Erkennen des zweiten Synchronisationsmarkers am zweiten Hörimplantat,
- Synchronisieren der Zeitschlitze des zweiten Hörimplantats mit den Zeitschlitzen der zweiten Verarbeitungseinheit anhand des Synchronisationsmarkers,
- Senden einer Bestätigungsnachricht vom zweiten Hörimplantat an die zweite Verarbeitungseinheit in einem zweiten Zeitschlitz der zweiten Verarbeitungseinheit,
- Abschließen der Herstellung der zweiten bidirektionalen drahtlosen Kommunikationsverbindung.

4. Ein binaurales Hörsystem nach einem der Ansprüche 1 bis 3,
wobei die Erfassungssequenz Folgendes umfasst: - Austausch der ersten ipsilateralen Datenpakete über die erste bidirektionale drahtlose Kommunikationsverbindung unter Verwendung des ersten und zweiten Zeitschlitzes an der ersten Verarbeitungseinheit für die jeweiligen Frames der Vielzahl aufeinanderfolgender Frames,
- Austausch der zweiten ipsilateralen Datenpakete über die zweite bidirektionale drahtlose Kommunikationsverbindung unter Verwendung des ersten und zweiten Zeitschlitzes an der zweiten Verarbeitungseinheit für die jeweiligen Frames der Vielzahl aufeinanderfolgender Frames,
- Übertragung eines dritten Synchronisationsmarkers von der ersten Verarbeitungseinheit zur zweiten Verarbeitungseinheit in einem dritten Zeitschlitz an der ersten Verarbeitungseinheit,
- Erkennung des dritten Synchronisationsmarkers an der zweiten Verarbeitungseinheit,
- Synchronisierung der Vielzahl von Zeitschlitzen an der zweiten Verarbeitungseinheit mit der Vielzahl von Zeitschlitzen an der ersten Verarbeitungseinheit basierend auf dem dritten Synchronisationsmarker,
- Übertragung einer Bestätigungsnachricht von der zweiten Verarbeitungseinheit zur ersten Verarbeitungseinheit in einem zweiten Zeitschlitz an der ersten Verarbeitungseinheit,
- Abschluss der Erfassungssequenz,
- Eintritt in den Normalbetrieb des binauralen Hörsystems.

5. Ein binaurales Hörsystem nach Anspruch 3 oder 4, wobei die Erfassung der bilateralen bidirektionalen drahtlosen Kommunikationsverbindung Folgendes umfasst:
- Verschiebung der Übertragung der zweiten ipsilateralen Datenpakete an der zweiten Verarbeitungseinheit mit einem Zeitschlitz zwischen den Frames der Vielzahl aufeinanderfolgender Frames mittels Wrap-Around,
- Verschiebung der Übertragung des Synchronisationsmarkers mit einem vorbestimmten Zeitschritt durch den dritten Zeitschlitz mittels Wrap-Around an der ersten Verarbeitungseinheit.

6. Ein binaurales Hörsystem nach Anspruch 5, wobei der vorbestimmte Zeitschritt weniger als 5 % der Länge eines Zeitschlitzes der Vielzahl von Zeitschlitzen des Rahmens beträgt.

7. Ein binaurales Hörsystem nach einem der Ansprüche 2 bis 6, wobei die Erfassung der bilateralen bidirektionalen drahtlosen Kommunikationsverbindung Folgendes umfasst: - Überwachung
des dritten Synchronisationsmarkers durch die zweite Verarbeitungseinheit , - vorübergehende Unterbrechung der zweiten bidirektionalen drahtlosen Kommunikationsverbindung durch die zweite Verarbeitungseinheit,
- Unterbrechung der Zeitschlitzverschiebung durch die zweite Verarbeitungseinheit bei gleichzeitiger Aufrechterhaltung der Übertragungsverschiebung des dritten Synchronisationsmarkers durch die erste Verarbeitungseinheit,
- Erkennung durch die erste Verarbeitungseinheit. Wenn sich die dritte Synchronisationsmarke im dritten Zeitschlitz der ersten Verarbeitungseinheit befindet,
- wird die dritte Synchronisationsmarke in der zweiten Verarbeitungseinheit erkannt und eine Bestätigungsnachricht von der zweiten Verarbeitungseinheit gesendet, - wird
die zweite bidirektionale drahtlose Kommunikationsverbindung vor Beendigung der Erfassungssequenz wiederhergestellt .

8. Ein binaurales Hörsystem nach einem der Ansprüche 1-4, wobei die Erfassungssequenz Folgendes umfasst:
- Aufrechterhaltung der Verbindung über die erste bidirektionale drahtlose Kommunikationsverbindung und Aufrechterhaltung der Verbindung über die zweite bidirektionale drahtlose Kommunikationsverbindung während der Erfassung der bilateralen bidirektionalen drahtlosen Kommunikationsverbindung.

9. Ein binaurales Hörsystem nach Anspruch 8, wobei die Erfassung der bilateralen bidirektionalen drahtlosen Kommunikationsverbindung Folgendes umfasst:
- Verschiebung der Übertragung der zweiten ipsilateralen Datenpakete an der zweiten Verarbeitungseinheit um einen vorbestimmten Zeitschritt zwischen den Frames der Vielzahl aufeinanderfolgender Frames mittels eines Wrap-Around-Verfahrens, wobei dieser vorbestimmte Zeitschritt kürzer als die Länge eines Zeitschlitzes ist,
- Überwachung der bilateralen bidirektionalen drahtlosen Kommunikationsverbindung durch die erste Verarbeitungseinheit auf eine Bestätigungsnachricht, die von der zweiten Verarbeitungseinheit als Reaktion auf die Erkennung des dritten Synchronisationsmarkers an der zweiten Verarbeitungseinheit gesendet wird.

10. Ein binaurales Hörsystem nach Anspruch 9, wobei die Zeitschrittverschiebung der Übertragung der zweiten ipsilateralen Datenpakete Folgendes umfasst:
- Vorwärtsbewegung der Übertragung um einen oder mehrere Umläufe in einer ersten Zeitrichtung durch die Vielzahl von Zeitschlitzen,
- vorübergehende Unterbrechung der Vorwärtsbewegung der Übertragung um einen oder mehrere Umläufe,
- Wiederaufnahme der Vorwärtsbewegung der Übertragung in einer zweiten Zeitrichtung durch die Vielzahl von Zeitschlitzen um einen oder mehrere Umläufe; wobei die erste und die zweite Zeitrichtung entgegengesetzt sind;
- Unterbrechung der Vorwärtsbewegung der Übertragung durch die zweite Verarbeitungseinheit als Reaktion auf die Übertragung der Bestätigungsnachricht,
- Synchronisierung der Vielzahl von Zeitschlitzen in der zweiten Verarbeitungseinheit mit der Vielzahl von Zeitschlitzen in der ersten Verarbeitungseinheit anhand des Synchronisierungsmarkers.

11. Ein binaurales Hörsystem nach einem der Ansprüche 2-10, wobei jede der Synchronisationsmarken mindestens Folgendes umfasst:
- eine vorbestimmte Binärsequenz,
- einen Zeitschlitzindikator , der angibt, welcher Zeitschlitz der Vielzahl von Zeitschlitzen in der ersten Verarbeitungseinheit die Synchronisationsmarke enthält.

12. Ein binaurales Hörsystem nach einem der Ansprüche 2 bis 11, wobei jedes Frame der Vielzahl aufeinanderfolgender Zeitframes mindestens vier nicht überlappende Zeitschlitze umfasst.

13. Ein binaurales Hörsystem nach Anspruch 11 oder 12, bei dem die Länge jedes der mindestens vier sich nicht überlappenden Zeitschlitze identisch ist.

14. Ein binaurales Hörsystem nach einem der vorhergehenden Ansprüche, wobei mindestens eine Teilmenge der ersten und zweiten ipsilateralen Datenpakete jeweils digitale Audiodaten wie digitale Echtzeit-Audiosignale umfasst; und/oder mindestens eine Teilmenge der bilateralen Datenpakete jeweils digitale Audiodaten wie digitale Echtzeit-Audiosignale umfasst.

15. Ein binaurales Hörsystem nach einem der Ansprüche 3-14, wobei jeder der ersten, zweiten und dritten Synchronisationsmarker eine eindeutige Paar-ID, beispielsweise einen eindeutigen Code oder eine eindeutige Nummer, zum Koppeln mindestens eines der folgenden Geräte umfasst:
das erste Hörgerät und das erste Hörimplantat,
das erste Hörgerät und das zweite Hörgerät oder das zweite Hörgerät und das zweite Hörimplantat.

16. Eine Erfassungssequenz gemäß einem gemeinsamen Kommunikationsprotokoll zum Aufbau von Verbindungen zwischen einem ersten Hörgerät und einem ersten Hörimplantat, zwischen einem zweiten Hörgerät und einem zweiten Hörimplantat sowie zwischen dem ersten Hörgerät und dem zweiten Hörgerät; wobei die Erfassungssequenz Folgendes umfasst:
- Aufbau einer ersten bidirektionalen drahtlosen Kommunikationsverbindung zwischen dem ersten Hörgerät und dem ersten Hörimplantat,
- Austausch erster ipsilateraler Datenpakete über die erste bidirektionale drahtlose Kommunikationsverbindung,
- Aufbau einer zweiten bidirektionalen drahtlosen Kommunikationsverbindung zwischen dem zweiten Hörgerät und dem zweiten Hörimplantat,
- Austausch zweiter ipsilateraler Datenpakete über die zweite bidirektionale drahtlose Kommunikationsverbindung; und als Reaktion auf den Aufbau der ersten und zweiten bidirektionalen drahtlosen Kommunikationsverbindungen:
- Aufbau einer bilateralen bidirektionalen drahtlosen Kommunikationsverbindung zwischen dem ersten und dem zweiten Hörgerät,
- Austausch bilateraler Datenpakete zwischen dem ersten Hörgerät und dem zweiten Hörgerät.

## Revendications

1. Un système auditif binaural comprenant :
un premier appareil auditif, un deuxième appareil auditif, un premier implant auditif et un deuxième implant auditif ;
le premier appareil auditif étant connecté au premier implant auditif via une première liaison de communication sans fil bidirectionnelle pour l'échange de premiers paquets de données ipsilatéraux,
le deuxième appareil auditif étant connecté au deuxième implant auditif via une deuxième liaison de communication sans fil bidirectionnelle pour l'échange de deuxièmes paquets de données ipsilatéraux,
une liaison de communication sans fil bidirectionnelle bilatérale étant connectée entre le premier et le deuxième appareil auditif pour l'échange de paquets de données bilatéraux ; ladite
liaison de communication sans fil bidirectionnelle bilatérale et lesdites première et deuxième liaisons de communication sans fil bidirectionnelles étant configurées pour fonctionner conformément à un protocole de communication commun qui comprend une pluralité de trames consécutives, chacune comprenant une pluralité d'intervalles de temps ;
ledit protocole de communication commun comprenant une séquence d'acquisition qui comprend : l'acquisition de la première liaison de communication sans fil bidirectionnelle à l'aide d'au moins une première unité de traitement du premier appareil auditif,
l'acquisition de la deuxième liaison de communication sans fil bidirectionnelle à l'aide d'au moins une deuxième unité de traitement du deuxième appareil auditif, les première et deuxième unités de traitement étant configurées pour acquérir la liaison de communication sans fil bidirectionnelle bilatérale en réponse aux acquisitions des première et deuxième liaisons de communication sans fil bidirectionnelles.

2. Un système auditif binaural selon la revendication 1, dans lequel la séquence d'acquisition comprend :
- le maintien d'au moins une des liaisons de communication sans fil bidirectionnelles première et deuxième pendant l'acquisition de la liaison de communication sans fil bidirectionnelle bilatérale.

3. Un système auditif binaural selon la revendication 1 ou 2, dans lequel :
l'acquisition de la première liaison de communication sans fil bidirectionnelle comprend :
- la transmission d'un premier marqueur de synchronisation de la première unité de traitement au premier implant auditif dans un premier intervalle de temps au niveau de la première unité de traitement,
- la surveillance de la pluralité d'intervalles de temps, au niveau du premier implant auditif, pour le marqueur de synchronisation,
- le glissement de la pluralité d'intervalles de temps au niveau du premier implant auditif avec des pas de temps prédéterminés entre les trames de la pluralité de trames consécutives à l'aide d'un schéma de bouclage,
- la détection du premier marqueur de synchronisation au niveau du premier implant auditif,
- la synchronisation de la pluralité d'intervalles de temps au niveau du premier implant auditif avec la pluralité d'intervalles de temps au niveau de la première unité de traitement sur la base du premier marqueur de synchronisation,
- l'achèvement de l'acquisition de la première liaison de communication sans fil bidirectionnelle,
- la transmission d'un message d'accusé de réception du premier implant auditif à la première unité de traitement dans un deuxième intervalle de temps au niveau de la première unité de traitement ;
L'acquisition de la seconde liaison de communication sans fil bidirectionnelle comprend les étapes suivantes : transmission d'un second marqueur de synchronisation de la seconde unité de traitement vers le second implant auditif dans un premier intervalle de temps au niveau de la seconde unité de traitement ;
surveillance des intervalles de temps, au niveau du second implant auditif, pour le second marqueur de synchronisation ;
glissement des intervalles de temps, au niveau du second implant auditif, avec des pas de temps prédéterminés entre les trames des trames consécutives à l'aide d'un schéma de boucle ;
détection du second marqueur de synchronisation au niveau du second implant auditif
; synchronisation des intervalles de temps au niveau du second implant auditif avec les intervalles de temps au niveau de la seconde unité de traitement en fonction du marqueur de synchronisation ;
transmission d'un message d'accusé de réception de la seconde unité de traitement vers la seconde unité de traitement dans un second intervalle de temps au niveau de la seconde unité de traitement ;
et achèvement de l'acquisition de la seconde liaison de communication sans fil bidirectionnelle.

4. Un système auditif binaural selon l'une quelconque des revendications 1 à 3, dans lequel la séquence d'acquisition comprend : - l'échange des premiers paquets de données ipsilatéraux via la première liaison de communication sans fil bidirectionnelle en utilisant les premier et deuxième intervalles de temps au niveau de la première unité de traitement des trames respectives de la pluralité de trames consécutives ;
- l'échange des deuxièmes paquets de données ipsilatéraux via la deuxième liaison de communication sans fil bidirectionnelle en utilisant les premier et deuxième intervalles de temps au niveau de la deuxième unité de traitement des trames respectives de la pluralité de trames consécutives ;
- la transmission d'un troisième marqueur de synchronisation de la première unité de traitement à la deuxième unité de traitement dans un troisième intervalle de temps au niveau de la première unité de traitement ;
- la détection du troisième marqueur de synchronisation au niveau de la deuxième unité de traitement ;
- la synchronisation des intervalles de temps au niveau de la deuxième unité de traitement avec les intervalles de temps au niveau de la première unité de traitement sur la base du troisième marqueur de synchronisation ;
- la transmission d'un message d'accusé de réception de la deuxième unité de traitement à la première unité de traitement dans un deuxième intervalle de temps au niveau de la première unité de traitement ;
- l'achèvement de la séquence d'acquisition ;
- le passage au fonctionnement normal du système auditif binaural.

5. Un système auditif binaural selon la revendication 3 ou 4, dans lequel l'acquisition de la liaison de communication sans fil bidirectionnelle bilatérale comprend :
- la transmission décalée des deuxièmes paquets de données ipsilatéraux à la deuxième unité de traitement avec un intervalle de temps entre les trames de la pluralité de trames consécutives en utilisant le retour à la ligne,
- la transmission décalée du marqueur de synchronisation avec un pas de temps prédéterminé à travers le troisième intervalle de temps en utilisant le retour à la ligne à la première unité de traitement.

6. Un système auditif binaural selon la revendication 5, dans lequel le pas de temps prédéterminé est inférieur à 5 % de la longueur d'un intervalle de temps parmi la pluralité d'intervalles de temps de la trame.

7. Système auditif binaural selon l'une quelconque des revendications 2 à 6, dans lequel l'acquisition de la liaison de communication sans fil bidirectionnelle bilatérale comprend :
- la surveillance, par la seconde unité de traitement, du troisième marqueur de synchronisation ;
- l'interruption temporaire de la seconde liaison de communication sans fil bidirectionnelle par la seconde unité de traitement ;
- l'interruption du décalage des intervalles de temps par la seconde unité de traitement tout en maintenant le décalage de transmission du troisième marqueur de synchronisation par la première unité de traitement ;
- la détection par la première unité de traitement. lorsque le troisième marqueur de synchronisation est placé dans le troisième intervalle de temps de la première unité de traitement,
- détecter le troisième marqueur de synchronisation de la deuxième unité de traitement et transmettre un message d'accusé de réception par la deuxième unité de traitement,
- réacquérir la deuxième liaison de communication sans fil bidirectionnelle avant la fin de la séquence d'acquisition.

8. Un système auditif binaural selon l'une quelconque des revendications 1 à 4, dans lequel la séquence d'acquisition comprend :
- maintenir la connexion via la première liaison de communication sans fil bidirectionnelle et maintenir la connexion via la deuxième liaison de communication sans fil bidirectionnelle pendant l'acquisition de la liaison de communication sans fil bidirectionnelle bilatérale.

9. Un système auditif binaural selon la revendication 8, dans lequel l'acquisition de la liaison de communication sans fil bidirectionnelle bilatérale comprend :
- le décalage de la transmission des deuxièmes paquets de données ipsilatéraux au niveau de la deuxième unité de traitement par un pas de temps prédéterminé entre les trames de la pluralité de trames consécutives à l'aide d'un schéma de bouclage, ledit pas de temps prédéterminé étant plus court que la durée d'un intervalle de temps,
- la surveillance, par la première unité de traitement, de la liaison de communication sans fil bidirectionnelle bilatérale pour un message d'accusé de réception transmis par la deuxième unité de traitement en réponse à la détection du troisième marqueur de synchronisation au niveau de la deuxième unité de traitement.

10. Un système auditif binaural selon la revendication 9, dans lequel le décalage temporel de la transmission des seconds paquets de données ipsilatéraux comprend :
- l'avancement de la transmission d'un ou plusieurs allers- retours dans une première direction temporelle à travers une pluralité d'intervalles de temps,
- l'interruption temporaire de l'avancement de la transmission d'un ou plusieurs allers-retours,
- la reprise de l'avancement de la transmission dans une seconde direction temporelle à travers une pluralité d'intervalles de temps pour un ou plusieurs allers-retours ;
les première et seconde directions temporelles étant opposées ;
- l'interruption de l'avancement de la transmission par la seconde unité de traitement en réponse à la transmission du message d'accusé de réception,
- la synchronisation des intervalles de temps de la seconde unité de traitement avec ceux de la première unité de traitement, sur la base du marqueur de synchronisation.

11. Un système auditif binaural selon l'une quelconque des revendications 2 à 10, dans lequel chacun des premier, deuxième et troisième marqueurs de synchronisation comprend au moins :
- une séquence binaire prédéterminée,
- un indicateur de créneau temporel indiquant quel créneau temporel parmi la pluralité de créneaux temporels de la première unité de traitement contient le marqueur de synchronisation.

12. Un système auditif binaural selon l'une quelconque des revendications 2 à 11, dans lequel chaque trame de la pluralité de trames temporelles consécutives comprend au moins quatre intervalles de temps non chevauchants.

13. Un système auditif binaural selon la revendication 11 ou 12, dans lequel la longueur de chacun des au moins quatre intervalles de temps non chevauchants est identique.

14. Un système auditif binaural selon l'une quelconque des revendications précédentes, dans lequel au moins un sous-ensemble des premier et deuxième paquets de données ipsilatéraux comprend des données audio numériques respectives telles que des signaux audio numériques en temps réel ; et/ou au moins un sous-ensemble des paquets de données bilatéraux comprend des données audio numériques respectives telles que des signaux audio numériques en temps réel.

15. Un système auditif binaural selon l'une quelconque des revendications 3 à 14, dans lequel chacun des premier, deuxième et troisième marqueurs de synchronisation comprend un identifiant de paire unique, tel qu'un code ou un numéro unique, pour apparier au moins un des éléments suivants :
le premier appareil auditif et le premier implant auditif,
le premier appareil auditif et le deuxième appareil auditif ; et le deuxième appareil auditif et le deuxième implant auditif.

16. Une séquence d'acquisition conforme à un protocole de communication commun pour établir les connexions respectives entre un premier appareil auditif et un premier implant auditif, entre un deuxième appareil auditif et un deuxième implant auditif, et entre le premier appareil auditif et le deuxième appareil auditif ; ladite séquence d'acquisition comprenant :
- l'acquisition d'une première liaison de communication sans fil bidirectionnelle entre le premier appareil auditif et le premier implant auditif,
- l'échange des premiers paquets de données ipsilatéraux via la première liaison de communication sans fil bidirectionnelle,
- l'acquisition d'une deuxième liaison de communication sans fil bidirectionnelle entre le deuxième appareil auditif et le deuxième implant auditif,
- l'échange des deuxièmes paquets de données ipsilatéraux via la deuxième liaison de communication sans fil bidirectionnelle ; et en réponse aux acquisitions des première et deuxième liaisons de communication sans fil bidirectionnelles :
- l'acquisition d'une liaison de communication sans fil bidirectionnelle bilatérale entre le premier et le deuxième appareils auditifs,
- l'échange de paquets de données bilatéraux entre le premier appareil auditif et le deuxième appareil auditif.
